# EUROPEAN PATENT APPLICATION

(11) **EP 4 782 024 A1**
(43) Date of publication of application: **29.07.2026**
(21) Application number: 25153905.2
(22) Date of filing: 24.01.2025
(51) Int. Cl.: A61M 1/36

(54) **TUBING CONNECTOR**

(71) Applicant: Fresenius Medical Care Deutschland GmbH, 61352 Bad Homburg (DE)
(72) Inventor: VENERONI, Alain, 61352 Bad Homburg (DE); CHIORINO, Carlo, 61352 Bad Homburg (DE); MASCHI, Lorenzo, 61352 Bad Homburg (DE)
(74) Representative: Lorenz Seidler Gossel Part. mbB

(57) **Abstract**

Tubing connector having an inlet for a liquid and an outlet for the liquid as well a first tubing connector element and a second tubing connector element, wherein the inlet and the outlet. The connector comprises at least one first pressure measurement chamber for measuring the pressure of the liquid which is present in said first wherein the first chamber is fluidly arranged between the inlet and the first tubing connector element or between the outlet and the second tubing connector element. The measuring surface is arranged in an angled orientation w.r.t to pumping plane. Further, a blood treatment device is adapted to accommodate the tubing connector.

## Description

The present invention relates to a blood treatment device, comprising a roller pump for pumping blood, a first pressure sensor arranged to measure the blood pressure upstream of the roller pump or downstream of the roller pump, optionally a second pressure sensor arranged to measure the blood pressure downstream or upstream of the roller pump. The present invention relates further to a pump and pressure measurement arrangement for the blood treatment device and relates further to tubing connector fitting physically and/or functionally in the pump and pressure measurement arrangement and relates further to a tubing set comprising the tubing connector.

In therapeutic treatments, such as hemodialysis, hemofiltration or hemodiafiltration, it is required to circulate blood to be treated from a patient to a blood treatment device, which can be a dialysis machine etc. and to further transport the treated blood back to the patient. In this context it is required to monitor the correct progress of the treatment including the pressure in the components which transport the patients' blood. Usually, roller pumps are used for this circulation of the blood, one example of which is disclosed in WO 2005/111424 A1. Roller pumps comprise a stator element and a rotor, the latter comprising one or more rollers. In use a tube is located between the stator element and the rollers and is pressed by means of the rollers to the stator elements which serve as pump bed.

WO 2014/147061 A1 discloses a connector for connecting an extracorporeal circuit with the tubing which is in contact with a roller pump. The connector is provided with a pressure sensor. WO 2012/166980 A2 is directed to a pressure measurement system for the use in blood circuits. The system comprises a pressure sensing pod which includes a flexible, moveable and fluid-impermeable diaphragm. A force measurement device is in engagement with a member of the diaphragm which is operative to move according to the movement of the diaphragm.

WO 2010/121819 A1 discloses a cassette for use in dialysis machines which comprises a number of chambers, channels and valves which are configured to control the fluid flow through the cassette.

It is an object of the present invention to provide a blood treatment device comprising a roller pump which has a simple construction, is easily to handle and allows an exact monitoring of the pressure in the blood treatment device.

This object of the invention is at least by the appended claims. The invention refers to a pump and pressure measurement arrangement, a blood treatment device, a tubing connector, a tubing set, and a system comprising pump and pressure measurement arrangement and the tubing connector, in particular a system the blood treatment device and the tubing set. Preferred embodiments of the invention are the subject-matter of dependent claims. The present invention also relates to the use of a blood transporting component in a blood treatment device according to the invention.

Even though the invention is described in relation to blood being the liquid that is pumped and/or of which the pressure is measured, also another liquid may be pumped and/or of which the pressure is measured. In particular the liquid may be a priming liquid, a substitution liquid, a reinfusion liquid, a physiological solution.

The present invention relates to an interface between a pumping and pressure measuring arrangement, in particular of a blood treatment device, and a tubing connector, in particular of a tubing set. Accordingly, the design of the pumping and pressure measuring arrangement is such that the tubing connector fits to the pumping and pressure measuring arrangement and vice-versa. Accordingly, the features of the pumping and pressure measuring arrangement are described in the specification and the features of tubing connector are described in the specifications.

In the following first the pumping and pressure measuring arrangement is described, followed by the description of the tubing connector. The figure section has a corresponding structure.

### The pumping and pressure measuring arrangement

The pump and pressure measurement arrangement which may be used in or may be integrated in a blood treatment device comprises a pump, in particular a roller pump for pumping a liquid, a pump bed which is configured to accommodate a pump-tubing, a first pressure measuring unit arranged to measure a pressure of the liquid, and an accommodation space for at least partially positioning a tubing connector connected to the pump-tubing, wherein the tubing connector further comprises a pressure measurement chamber adapted to measure the pressure with the pressure measuring unit.

It is noted that the pump bed may also be considered forming a part of the pump. The pump may comprise a stator being connected to a motor for driving a rotor to which at least two rollers of the roller pump are attached. In pumping operation, the pump-tubing is positioned in the pump bed and the rollers when running over the pump-tubing squeeze the tube pushing a liquid inside the tube forward. The pump-tubing may have a loop or an omega shape when inserted in the pump bed. The design of such a peristaltic pump is well known to a person skilled in the art.

"Pump-tubing" may have the meaning of a compressible tubing that is configured to be squeezed by the rollers of the peristaltic pump.

"Accommodation space" may have the meaning of a space particularly designed in dimensions and/ or structuring so that the tubing connector fits thereto. This distinguishes the "accommodation space" from any other space between a pump and pressure measuring unit where there is no structural and/or functional relation between a pump and a pressure measuring unit. An example for such a non-structural and/or functional relation may be for example present if the pressure measuring chamber on the tubing set is connected through a flexible tube to the pumping tube and, accordingly the position of the pressure measuring unit on the extracorporeal blood treatment device is not determined by the position of the pump on the extracorporeal blood treatment device. Accordingly, the tube connector may be a building unit in which none of the pumping connections are connected to the first pressure measurement chamber via a flexible tubing, but the connection is provide through a formstable channel. In particular, the tube connector may consist of a single molded element and a membrane.

The accommodation space may be located between the pump and the first pressure measuring unit or adjacent to the pump and the first pressure measuring unit.

"Between" may have the meaning that the tubing connector when positioned on the accommodation space substantially fills the complete space between the pump and the pressure measuring unit and/or no other components may be positioned therebetween. Accordingly, "adjacent" may have the meaning that pressure measuring unit is in immediate vicinity substantially not leaving sufficient space for any other component in between. As it will be apparent from the following the differentiation between "between" and "adjacent" is the orientation of the measuring plane of the pressure measuring unit.

With such an accommodation space it may be possible to position the tubing connector in a single manual positioning operation of the tubing connector for both functionalities, i.e. pumping and pressure measurement. It may further allow that with a single fixation mechanics the tubing connector may be fixed in the pump and at the same time the pressure measurement chamber of the tubing connector is positioned with an orientation appropriate for being coupled with the pressure measuring unit. In other words, the pump or the pump bed may be configured for positioning of the tubing connector providing with this positioning a parallel or substantially parallel orientation of a measuring surface of the measuring chamber of the tubing connector to a measuring surface of the pressure measuring unit.

The accommodation space may have a width between the pump and the pressure measuring unit of less than 5 cm, preferably less than 3 cm. These dimensions may have the advantage of providing the functionalities at reasonable manufacturing costs referring to the pump, the pressure measuring unit and the tube connector as both assembly within a very small space and also small sized building elements may drive costs.

At least part of the first pressure measuring unit may be moveable from and towards the accommodation space and/or from and towards the tubing connector and/or from and towards the pump bed.

The movable part of the first pressure measuring unit may be a measuring surface of the first pressure measuring unit.

Such a design of the accommodation space may allow for an automatic coupling of the measuring surface of the measuring chamber of the tubing connector to the measuring surface of the pressure measuring unit. At the same time, it may allow for positioning of the tubing connector in the accommodation space without physical interaction with the measuring surface of the pressure measuring unit and the measuring surface of the pressure measuring unit may not experience any friction or deformation upon positioning of the tubing connector.

The movable part of the pressure measuring unit may be, alternatively or additionally to the measuring surface, a fixing and moving element configured to hold the tubing connector and to move the tubing connector from and towards measuring surface of the pressure measuring unit.

In an alternative embodiment the fixing and moving element is not part of the pressure measuring unit, but an independent building block of the pump and pressure measuring unit.

In an alternative embodiment the fixing and moving element is not part of the pressure measuring unit, but an independent building block of the pump and pressure measuring unit and no part the pressure measuring unit is movable from and towards the accommodation space and/or from and towards the tubing connector.

A direction of the movement from and towards the pump bed and/or the tubing connector may be in the plane which is defined by the pump bed or in a plane parallel thereto or in a plane which has an angle with respect to the plane of the pump bed, preferably an angle of 90°.

In particular, the movement of the part of the first pressure measuring unit may be a in direction parallel to a symmetry axis of the pump bed and parallel, wherein the pump bed has a single opening for positioning the tube connector. The movement of the part of first pressure measuring unit may be laterally offset from the symmetry axis.

The first pressure measuring unit may have a measuring surface which is located in the same plane as the plane of the pump bed, in a plane parallel thereto or on a plane which is arranged at an angle with respect to the plane of the pump bed, preferably at an angle of 90°.

A bottom of the accommodation area may be in the same plane as the pump bed or recessed with regard to the pump bed and/or wherein the lowest part of the first pressure measuring unit is in the same plane as the pump bed or recessed with regard to the pump bed.

"Bottom" may have the meaning of a surface onto which the tubing connector is positioned and "lowest" may have the meaning of the direction vertical to the pump bed plane, wherein the closer an element is arranged to an extracorporeal blood treatment device housing or the closer to a motor for driving the pump, the lower it is positioned.

A center of the measuring surface may lie within or substantially within the plane of the pump bed or within a plane of a pump tube loop formed by the pump-tubing.

With such an arrangement the measuring surface may extend to both sides of the plane of the pump bed or the plane of the pump tube loop. With this design it may be possible to provide a pressure measuring unit that is not or only less elevated over the pump or pump bed wall. This arrangement may allow for a more symmetrical design of the tube connector as its inlet, pump-tubing connector elements and outlet may be provided on substantially the same plane allowing for simplified manufacturing technology, tubings that are guided in a plane, in particular a surface plane of the device in which the pump and pressure measurement arrangement is implemented, thereby allowing an easier handling by the user.

The arrangement may comprise a second pressure measuring unit for measuring a second pressure, wherein preferably the second pressure measuring unit is located alongside the first pressure measuring unit and preferably parallel thereto.

The first and the second pressure measuring unit each may have a measuring surface, which surfaces being preferably arranged in the same plane.

Such a design may allow to basically use the same mechanics for the two pressure measuring units, in particular for the movement towards and from the accommodation space. At the same time this symmetry may be reflected on the tubing connector allowing potentially for a cheaper manufacturing technology such as using less complex molds. Also, the controlling of the coupling for conducting the pressure measurement may be similar and the same methods may be applied to both pressure measurement units.

The first pressure measuring unit and the second pressure measuring unit may have at least partially or completely the same features. The only difference may be the physical positioning, the pressure measurement chamber with which they interact and the controls for which the signals of the measuring unit are uses. Thus, any description and/or feature that is described with respect to the first or the second pressure measuring unit may also be present for the respective other unless specifically pointed-out in this description. Where a "pressure measuring unit" is described this may be applied to the first pressure measuring unit only or the second pressure measuring unit only or to both. The first pressure measuring unit may be configured to measure the pressure upstream of the pump and the second pressure measuring unit may be configured to measure the pressure downstream of the pump or vice versa.

The pressure measuring unit may comprise a flat, ring-shaped protection element surrounding the measuring surface of the pressure measuring unit and, preferably, wherein in a pressure measuring position of the pressure measuring unit a slit between the protection element and the tubing connector is smaller than in a not-pressure measuring position.

Due to the arrangement of the two (or more) pressure measuring units and the respective pressure measurement chambers on the tubing connector it may be possible to measure the pressure before, i.e. upstream, and after, i.e. downstream of the roller pump and thus to provide a very exact monitoring of the pressures at different locations. Further it is possible to provide a comparison unit which is configured to compare the pressure values which are measured by the two pressure sensors and in case a difference between the values exceeds a threshold triggers a warning message may be displayed or a stop of the treatment may be initiated.

The arrangement may comprise one or more pins which are adapted to interact with the tubing connector and which are moveable towards and backwards to and from the accommodation space.

The pin or the pins may be rotatable between a first and a second position wherein in the first position the pins are adapted to fix the tubing connector in the accommodation space in its insertion direction and in the second position the pin or the pins are adapted not to fix the tubing connector in the accommodation space in its insertion direction.

The pin or the pins may be provided in particular in an embodiment in which the measuring surface is located in the same plane as the plane of the pump bed, or in a plane parallel thereto. The pin or the pins may provide the fixation of the tubing connector such that a movement in the direction of the movement of the part of the pressure measuring unit is hindered. This may allow for measuring the pressure in the pressure measurement chamber through the coupling of the pressure measuring surfaces. The arrangement in which in which the measuring surface is arranged on a plane which is arranged at an angle with respect to the plane of the pump bed, preferably at an angle of 90° no such pin or pins may be present.

The pump bed may be formed by a wall which at least in part surrounds the pump bed. The rollers of the pump may be oriented with their rotation axis parallel to the axis of the rotator and the squeezing force of the rollers may be directed radially to the rotor rotation. The pump bed in which the pump-tubing may be positioned thus is formed by the pump bed wall. While the pump bed wall on one side is basically defined by the requirements of the peristaltic pump action, for example the pump bed forms at least a part of a circle, there must be provided at least one open part or opening in the pump bed wall through which the tubing runs when the pump-tubing is arranged in the pump bed. In the area of the of the open space the wall may protrude in form a protrusion from a circular pump bed wall course.

In other words, the protrusion may comprise an open part which is located between the pump bed and the accommodation space, wherein the open part defines a space through which the pump-tubing runs when connected to the arrangement.

The protrusion may comprise two, optionally symmetrical legs, extending from the circular pump bed wall in the direction of the accommodation space.

The protrusion may have at least one portion where the tubing connector abuts in an operation condition in a direction along the normal of a plane of the measuring surface and in which the pressure measuring unit is in contact with the tubing connector.

"Operation condition" may have the meaning of a device setup condition in which the measuring surface of the pressure measuring unit is in physical contact to a membrane (or measuring surface of the tubing connector) of the tubing connector allowing for transferring the pressure insight the pressure measurement chamber of the tubing connector onto the measuring surface of the pressure measuring unit. This operating condition this basically corresponds to a pressure measuring position.

The coupling between the measuring surface of the pressure measuring unit and a membrane (or measuring surface of the tubing connector) of the tubing connector may be reached by bringing the two surfaces into contact, thereby squeezing the air out of the space between the two surfaces. Once the air is squeezed out the two surfaces may be adhere to each other and upon pressure change inside the pressure measurement chamber the measuring surface of the pressure measurement chamber moves in a direction of the normal of the measuring surface of the pressure measuring unit and through the coupling the measuring surface of the pressure measuring unit also moves. This movement may then be detected by respective sensors attached or sensitive to the movement.

The coupling may be reached only be the adhesion forces without any other measures. In an alternative embodiment the coupling may in addition be reached by a vacuum generating unit for removing the air between the two surfaces.

The arrangement may comprise an undercut which is arranged to fix the tubing connector by form fit, wherein preferably the undercut is part of the pressure measuring unit and/or part of the pump bed and/or of the protrusion.

The portion may comprise one or more recesses and/or protrusions for fixing the tubing connector.

The accommodation space for locating a tubing connector may be configured so that the tubing connector may only be inserted and removed to and from the accommodation space in exactly one direction, wherein preferably said direction is perpendicular to the surface of the accommodation space.

The arrangement may provide no guiding pins or rods for positioning the tubing connector. In particular, the arrangement may not provide three or more guiding pins or rods. Accordingly, the tubing connector may have no openings or through-holes for accommodating such guiding pins or rods. In particular, the tubing connector may not have three or more such openings or through-holes. The arrangement may guide the positioning of the tubing connector only through its shape, for example through the shape of the opening in the pump bed wall or in the protrusion, the space between pump and the pressure measuring unit or an undercut or undercuts. The tubing connector may have accordingly an outer shape that fits into the shape of the arrangement. The pressure measuring unit may comprise a mechanical unit configured for moving the measuring surface of the pressure measuring unit towards the connector for coupling the measuring surface of the pressure measuring unit and the connector. The mechanical unit may be connected to a motor and a controller for positioning the measuring surface of the pressure measuring unit. The pressure measuring unit may additionally or alternatively comprise one or more pneumatic element for moving the measuring surface of the pressure measuring unit towards the connector for coupling the measuring surface of the pressure measuring unit and the connector. The pneumatic unit may be connected to a pump and a controller for positioning the measuring surface of the pressure measuring unit.

The pumping and pressure measuring arrangement with the inserted tubing connector, optionally with pump-tubing and tubings connected to the inlet and outlet of the tubing connector:
The pumping and pressure measuring arrangement may comprise a tubing connector which is located in the accommodation space.

The tubing connector may be the tubing connector as described below under the headline "tubing connector" and as described with reference to the drawings.

The tubing connector of this arrangement may have an outlet for a liquid, an inlet for a liquid as well as a first tubing connector element and a second tubing connector element, wherein each the inlet and the outlet are both configured to be fluidly connected with an tubing, in particular a tubing guiding the fluid away and/or towards the pump and wherein the first and the second tubing connector elements are both configured to be fluidly connected to a pump-tubing, in particular a pump-tubing configured for pumping fluid in a peristaltic pump and forming a loop, in which the pump-tubing is connected to the first tubing connector with a first end and is connected to the second tubing connector with a second end, wherein the tubing connector comprises at least one first blood pressure measurement chamber for measuring the pressure of the liquid which is present in said first chamber, said first pressure measurement chamber being in contact with the the first pressure measuring unit in the operating condition, said first pressure measurement chamber comprising a first flexible membrane which covers an opening in said first pressure measurement chamber, wherein the inlet and the first tubing connector element or the outlet and the second tubing connector element are fluidly connected to said first chamber.

The tubing connector may be no integral part of the pressure measuring unit and/or the tubing connector may be releasable fixed to the accommodation space.

The pressure measuring unit or part thereof may be moveable towards and backwards to and from the membrane of the first pressure measurement chamber of the tubing connector.

The tubing connector may be fixed by snap fit and/or force fit in the accommodation space.

The pin or pins may be located and configured to release the connector in part of fully from its operating position in the accommodation space, and in particular released in part of fully from a form-fit position, when the pin or pins are moved towards the tubing connector, wherein preferably the tubing connector comprises at least one opening for accommodation the pin or two openings for accommodating two pins.

The pin or the pins may be arranged to fix the tubing connector in all three spatial directions in at least one position of the pin or the pins.

The tubing connector may comprise one or more recesses and/or protrusions and/or the pump and/or pressure measurement arrangement may have recesses and/or protrusions which interact when the tubing connector is positioned in the accommodation space, in particular on the protrusion of the wall of the pump bed, in particular on the one portion of the protrusion against which the tubing connector abuts in an operation condition in a direction along the normal of a plane of the measuring surface and in which the pressure measuring unit is in contact with the tubing connector and/or on the pressure measuring unit, in particular on a wall of the pressure measuring unit. Such an interaction may allow for a pre-fixation of the tubing connector in the accommodation space such that the tubing connector does not fall off.

The connector may have play on at least one side after its insertion and before operation of the arrangement and has no play in operation of the arrangement. In other words, when the tubing is initially inserted in the accommodation space, there may still be some lateral movement possible. This allows for an insertion of the tubing connector into the accommodation space with no manual force or at least a minor manual force. In addition, as described above, the measuring surface of the pressure measuring unit may not be impacted through friction or force when the tubing connector is inserted. This may further allow for a better control of coupling of the measuring surfaces, i.e. the membrane of the pressure measurement chamber and the measuring surface of the pressure measuring unit. As described above, this may be necessary to squeeze the air out of the area between the two measuring surfaces in a controlled and reproducable manner. In an operation condition there is no play anymore. This means that the measuring surfaces are in physical contact to each other and upon movement of the pressure measurement chamber surface the pressure measuring surface of the pressure measuring unit moves accordingly. The coupling between the two measuring surfaces may only be based on adhesion forces of the materials.

The pumping and pressure measuring arrangement may comprise no means for generating vacuum between the membrane and the pressure measuring unit.

The pumping and pressure measuring arrangement may comprise the first and the second pressure measuring unit and the tubing connector may comprise a first and a second blood pressure measurement chamber. Each of the first and second blood pressure measurement chambers may be provided with a membrane or pressure measuring surface, the membrane or pressure measuring surface of the first pressure measurement chamber may couple and/or interact with the first pressure measuring unit and the membrane or pressure measuring surface of the second pressure measurement chamber may couple and/or interact with the second pressure measuring unit or vice versa.

The membrane or pressure measuring surface of the first pressure measurement chamber and the membrane or pressure measuring surface of the second pressure measurement chamber may be two separate sheets or foils or any other flexible material or plastic being air tight and suitable to be deformed by the pressure inside the pressure measurement chamber, in particular may be no single foil covering both pressure measurement chambers and being welded to a rim of the both pressure measurement chambers.

The first blood measuring chamber may be located upstream of the roller pump and the second blood pressure measurement chamber may be located downstream of the roller pump.

The measuring surfaces of the pressure measuring units may be parallel to the membranes of the pressure measurement chambers.

The first pressure measurement chamber may comprise the inlet and the first tubing connector and the second pressure measurement chamber may comprise the outlet and the second tubing connector.

The first and second tubing connector elements may each have a longitudinal axis, wherein the longitudinal axes of both tubing connector elements preferably run crosswise when projected in a plane.

The first and second tubing connector may be fluidly connected through channels with the first and second pressure measurement chambers.

The thorough channels may run along the longitudinal axis and provide a straight connection between a port of the tubing connector element to which the pump-tubing is or may be connected and the pressure measurement chamber. This may allow to provide a small sized tubing connector requiring less material and also allowing the pressure generated in the pump being directly transferred into the pressure measuring chamber without pressure changes due to bending or angled channels through which the liquid is flowing.

The pump bed may have a first and a second end portion wherein the longitudinal axis of the first and second tubing connector element each aligns with said first and second end portion, respectively, in particularly aligns substantially tangential with the end portions of the pump bed.

A pump-tubing may be arranged in the pump bed.

The pump-tubing may run loop shaped and may have two end portions, each of which ending in or adjacent to the accommodation space.

The two end portions of the pump-tubing may be fluidly connected to the first and second tubing connector element of the connector, respectively. The two end portions of the pump-tubing may be physically connected to the first and second tubing connector elements of the connector, respectively. In particular, the connection may be a gluing, heat welding, laser welding or any other bonding technology connection.

The pump-tubing loop may be arranged in a first plane and the membrane or measuring surfaces of the first pressure measurement chamber and/or of the second pressure measurement chambers may be in the same plane or in a plane which is arranged parallel to said first plane or substantially in the same plane or substantially parallel to said first plane.

In accordance with a further embodiment said pump-tubing may be arranged in a first plane and the membrane or measuring surfaces of the first pressure measurement chamber and/or of the second pressure measurement chambers may be in a second plane which is arranged at an angle to said first plane and which is preferably perpendicular to said first plane.

It is noted "parallel" and "substantially parallel" may be used exchangeable in relation to the pump-tubing loop. The reason is that the pump-tubing loop may be slightly inclined with respect to a plane formed by the membrane or measuring surfaces of the first pressure measurement chamber and/or of the second pressure measurement chambers due to positioning of the first tubing connector element and the second tubing connector element one slightly higher than the other. This shall not be considered a deviation from a parallel orientation. The same applies when a perpendicular orientation is described. This is described in more detail in the description of the drawings. It is further noted that the term "first plane" in which the pump-tubing may be arranged encompasses the case that the pump-tubing is actually in one plane as well as the case that the pump-tubing is inclined as mentioned before. In the latter case the "frist plane" is a projected plane of the pump-tubing.

The arrangement may comprise one or more doors, which in the closed position cover the pump, the pump bed, and at least partially the tubing connector. The door or the doors may in addition cover the complete tubing connector and the pressure measuring unit.

In the opened position the door may be configured to allow access to at least one, preferably to all of said components, in particular to the components covered in the closed position.

The door or the doors may be configured to contact the tubing connector when closed and/or may be adapted to withstand the force generated for coupling between the pressure measuring unit and the tubing connector.

### Blood treatment device

A blood treatment device, in particular an extracorporeal blood treatment device, may comprise at least one pumping and pressure measuring arrangement as described above.

Further, the blood treatment device may comprise at least one pumping and pressure measuring arrangement with a tubing connector as described above inserted in the pumping and pressure measuring device

The blood treatment device may be a dialysis device, in particular a hemodialysis, a hemofiltration, a hemodiafiltration or an ultrafiltration device.

The pump of the pumping and pressure measuring arrangement may be a blood pump of the blood treatment device.

The pressure measuring unit or the pressure measuring units of the pumping and pressure measuring arrangement may be configured to measure the pressure in an arterial tubing configured to guide blood from a patient towards a filter, in particular a dialyzer or an ultrafiltration filter. The arterial tubing may consist of at least two sections, one being connected to the inlet and the second being connected to an outlet of the tubing connector.

The blood treatment device may further comprise at least one, more than one or all of the following components, a controller for controlling the operation of the blood treatment device, in particular for controlling the pump and the pressure measuring unit, in particular for controlling the pumping action of the pump, the coupling of the tubing connector to the pressure measuring unit, the data acquisition of the pressure measuring unit, a monitor for displaying information, in particular the values measured by the pressure measuring unit or the pressure measuring units, a system for online generation of dialysis fluid from at least two concentrates and water.

The pump and pressure measuring unit may be provided on a front side of the blood treatment device. There may be more than one pump and pressure measuring unit provided with the blood treatment device, a first one for pumping blood a second one for pumping another fluid such as substitution fluid and/or blood reinfusion fluid.

The blood treatment device may further comprise a venous pressure measuring unit for measuring a venous pressure which is a pressure inside a venous tubing for guiding the blood from the filter back into the patient. The venous pressure measuring unit may be mechanically independent from the pump and the pressure measuring unit. The venous pressure measuring unit may comprise a port of the front of the blood treatment device configured to be connected to an air-filled branch line of the venous tubing and that may measure the pressure inside the blood treatment device via the air pressure inside the air-filled branch line.

A tubing connector or the tubing connector as described above may have an inlet for a liquid and an outlet for the liquid as well a first tubing connector element and a second tubing connector element, and having at least one pressure measurement chamber fitting physically and/or functionally in an pumping and pressure measuring arrangement as described above.

In particular the tubing connector may have a shape to fit in the opening in the pump bed and to fit to the accommodation space. Further, the tubing connector may have a membrane or measuring surface adapted to be coupled to the measuring surface of the pressure measuring unit.

A tubing set may comprise the tubing connector as described above, having a pump loop being connected to the first and second pump loop connector and a first tubing being connected to the inlet and a second tubing being connected to the outlet. The first tubing may be a first arterial tubing section for guiding blood from the patient to the tubing connector and the second tubing section may be a second arterial tubing section for guiding blood from the tubing connectors towards the filter.

The tubing set may further comprise a venous tubing for guiding the blood from the filter back into the patient. The tubing set may comprise on the arterial or the venous tubing a first flexible branch line for conveying fluid into the respective tubing.

The tubing set may comprise on the arterial or the venous tubing a second flexible branch line for conveying fluid into the respective tubing, the first branch line being connected to the arterial tubing and the second branch line being connected to the venous tubing.

The first and second branch line may be attached irreversibly to the tubing, in particular by gluing using an adhesion medium, heat gluing by melting and solidifying the material or by laser gluing where heat is generated by a laser light absorbing agent in the tubing and/or branch line material.

At a port of the arterial tubing, where the first branch line is connected to the arterial tubing, a check valve may be provided allowing a flow into the arterial tubing from the first branch line and blocking a flow from the arterial tubing into the first branch line.

At a port of the venous tubing, where the second branch line is connected to the venous tubing, a check valve may be provided allowing a flow into the venous tubing from the second branch line and blocking a flow from the venous tubing into the second branch line.

The first and second branch line may be connected at ends not connected to the arterial tubing and not connected to the venous tubing with each other, in particular via a Y-piece or a T-piece.

The arterial tubing or tubing sections and the venous tubing may comprise more than one, two or three tubing section being connected to each other through functional or non-functional elements. Such elements may be formed of hard plastic parts to which the tubings are attached, e.g. by gluing. Such elements may be one or more of an air separation chamber, in particular a venous air separation chamber provided on the venous tubing or an arterial venous air separation chamber provided on the arterial tubing, an infusion port, in particular an infusion port having a valve assembly or a septum. Any air separation chamber may have a substantially cylindrical or a conical shape, in particular formed by a hollow body and made of a rigid plastic having a stable shape to which the venous tubing is fixed. A branch line may be connected to an upper area of the air separation chamber, configured to be connected to the venous pressure sensor of the blood treatment device described above. At the lower end of the air separation chamber an opening may be provided for blood to exit the air separation chamber, in particular towards the patient. Within the hollow body a conical or a truncated conical clot catcher may be provided in particular within the blood flow entering the hollow body and existing the blood flow.

The tubing set may be no extracorporeal blood cassette.

The tubing set may be an extracorporeal circuit which may be connected to a patient for removing and transferring back blood from and to the patient.

"Tubing" in this description has a meaning of a flexible, non-rigid tubing, in particular an at least in part bendable tubing. It is to be distinguished from a channel made of a formstable plastic. Such formstable plastic channels are in particularly regularly provided on extracorporeal blood cassettes.

At this point it is pointed out that the terms "one" and "a/an" do not necessarily refer to exactly one of the elements, although this is a possible version, but can also denote a plural of the elements. Likewise, the use of the plural also includes the presence of the element in question in the singular and, conversely, the singular also includes several of the elements in question.

Further, all of the features of the invention described herein may be claimed in any combination or in isolation from one another.

Further details and advantages of the invention will be explained in more detail with reference to an example embodiment shown in the drawing.

### Tubing Connector:

The present invention further relates to a tubing connector as described above. The tubing connector has an inlet for a liquid and an outlet for the liquid as well a first tubing connector element and a second tubing connector element, wherein the inlet and the outlet are both configured to be fluidly connected with a tubing.

Said tubing may be an extracorporeal blood tubing used during dialysis treatment for conveying blood from the patient towards the dialyzer.

The term "liquid" refers for example to blood, physiological saline solution, substitution fluid, reinfusion fluid, RO water and any other physiological fluid as well as a combination of any of the aforementioned liquids, such as blood to which a substitution fluid etc. has been added.

The first and the second tubing connector elements may both be configured to be fluidly connected to a peristaltic pump-tubing. A pump-tubing according to the present invention is characterized to be suitable for use in a peristaltic pump. It is made of soft material so that the rollers or actuators of the peristaltic pump can fully compress the tubing. Said material is usually softer than e.g. the material of the other tubing, such as an extracorporeal tubing of a dialysis machine. The diameter of the pump-tubing may be larger than that of another different tubing such as an extracorporeal tubing of a dialysis machine.

The tubing connector comprises at least one first pressure measurement chamber for measuring the pressure of the liquid which is present in said first chamber which may have a chamber wall with an opening for the pressure measurement, said first chamber may comprise a first flexible membrane which covers said opening in total or in part wherein the first chamber may be fluidly arranged between the inlet and the first tubing connector element or between the blood outlet and the second tubing connector element. Said opening may be circular, however, other shapes of the opening are also conceivable and covered by the present invention. The first flexible membrane may be considered the measuring surface of the pressure measurement chamber.

According to an embodiment the inlet comprises a first elongated channel having a first longitudinal axis and the outlet comprises a second elongated channel having a second longitudinal axis and/or the first tubing connector element comprises a third elongated channel having a third longitudinal axis and the second tubing connector element comprises a fourth elongated channel having a fourth longitudinal axis.

The channels may be straight or curved and/or may be cylindrical or conical in shape.

The longitudinal axes of the first and second channel may cross outside the tubing connector and the longitudinal axes of the third and fourth channel may cross inside the tubing connector.

In accordance with a further embodiment, the first longitudinal axis of the first channel and the second longitudinal axis of the second channel lie on the same straight line or lie on straight lines which are parallel to each other.

The longitudinal axes of the first and second channel may cross on one side of the pressure measurement chamber and the longitudinal axes of the third and fourth channel may cross on the opposite side of the pressure measurement chamber. "Crossing" means that they intersect when viewed from above or projected in the same plane.

In an embodiment the first longitudinal axis and the second longitudinal axis are not parallel to each other and/or the third longitudinal axis and the fourth longitudinal axis are not parallel to each other.

In accordance with a further embodiment the first longitudinal axis and the second longitudinal axis form an angle with each other which is in the range between 75° and 105°, preferably between 85° and 95° and most preferred 90° and / or the third longitudinal axis and fourth longitudinal axis form an angle with each other which is in the range between 75° and 105°, preferably between 85° and 95° and most preferred 90°.

In accordance with a further embodiment the first longitudinal axis and the second longitudinal axis and, optionally the fifth longitudinal axis, run in parallel.

The third and fourth longitudinal axes may be parallel to a plane of the membrane. The first and the second longitudinal axes may also run parallel to a plane of the membrane.

In an embodiment, the first axis and the second axis run parallel to a plane of the membrane, but a straight line, passing through the first longitudinal axis and the second longitudinal axis and perpendicular thereto does not cross the membrane.

In an embodiment the membrane is offset from the crossing of the first and second tubing connector elements in a direction of one of the third and fourth longitudinal axes. In other words, the membrane does not cover an area of the crossing of the first and second tubing elements.

Alternatively, the third and fourth longitudinal axes intersect a plane of the membrane. In this case the first and second longitudinal axis may intersect a plane of the membrane.

The first and fourth longitudinal axes may be parallel to each other. The first and the third axes may run perpendicular to each other. Alternatively or additionally, the second and third longitudinal axes may be parallel to each other. The second and fourth longitudinal axes may run perpendicular to each other.

The tubing connector may comprise a first pressure measurement chamber being fluidly connected to the inlet and the first tubing connector and a second pressure measurement chamber being fluidly connected to the outlet and the second tubing connector.

Alternatively, the tubing connector may comprise a first pressure measurement chamber being fluidly connected to the outlet and the second tubing connector element and a second pressure measurement chamber being fluidly connected to the inlet and the first tubing connector element.

The tubing connector may further comprise a further fluid inlet having a channel with an opening into said first pressure measurement chamber. This further fluid inlet may be a Heparin line connector. Alternatively, said further fluid inlet is fluidly connected to the second pressure measurement chamber of the tubing connector. There may also be provided two further fluid inlets, the first being connected to the first pressure measurement chamber, the second being connected to the second pressure measurement chamber.

This further fluid inlet may have a fifth longitudinal axis.

Said fifth longitudinal axis of the channel of the further fluid inlet may be parallel to the second and third longitudinal axis, and preferably the fifth, the second and the third longitudinal axis lie in a same plane. Said fifth longitudinal axis may be parallel to the first and third axis and/or may lie in the same plan as the first and third axis. The foregoing may also apply for any additional inlet of the tubing connector.

The further fluid inlet may be located separate from the inlet and/or the outlet of the tubing connector or the further fluid inlet and one of the inlet and the outlet of the tubing connector are formed by a common, i.e. single element.

In particular the further fluid inlet may be located separate from the inlet and/or the outlet of the tubing connector. This may provide additional space and may facilitate the assembly of said further fluid inlet line to the tubing connector.

The tubing connector may comprise a groove or any other fixing element which is configured to hold a tubing which is configured to be fluidly connected to the further fluid inlet.

The tubing connector may comprise a push tab which allows the user to exert force on the tubing connector in order to arrange the tubing connector in an accommodation space. Said push tab may be located directly next to said groove which is configured to hold the tube which is connected with the further fluid inlet.

Providing as many channels with a parallel orientation may simplify the respective design of the molding tool to manufacture the tubing connector, as the pin movements for the injection molding mold are parallel to each other. The same may apply for an in-plane design for as many channels as possible as the mold may have a less bulky design and may require fewer undercuts.

In accordance with a further embodiment of the invention the fifth longitudinal axis intersects the plane of the membrane or is parallel to the plane of the membrane or is tangential to the first pressure measurement chamber, wherein the first pressure measurement chamber has a circular base.

In accordance with a further embodiment the tubing connector does not comprise any other connector but the inlet, the outlet, the first tubing connector element, the second tubing connector element and the further fluid connector. "Connector" has here the meaning of a port through which fluid may be guided into or out of the tubing connector.

In an embodiment the inlet and the first tubing connector element end in the first pressure measurement chamber or the outlet and the second tubing connector element end in the first pressure measurement chamber.

In an embodiment the inlet and the first tubing connector element end in the first pressure measurement chamber and the outlet and the second tubing connector element do not end in the first pressure measurement chamber.

In an embodiment the inlet and the first tubing connector element end in the first pressure measurement chamber and the outlet and the second tubing connector element do not end in the first pressure measurement chamber but end in the second pressure measurement chamber.

In an embodiment the inlet and the first tubing connector element do not end in the first pressure measurement chamber but in the second pressure measurement chamber and the outlet and the second tubing connector element end in the first pressure measurement chamber.

Further, at least two of the first, the second, the third, and the fourth longitudinal axes may form a plane, which plane is the same plane as that of the membrane of the first pressure measurement chamber or a parallel plane or which extends at an angle with respect to a plane of the membrane of the first pressure measurement chamber, in particular perpendicularly.

The longitudinal axis of at least one of the inlet, the outlet, the first tubing connector element and the second tubing connector may extend at an angle with respect to a plane of the membrane of the first pressure measurement chamber, in particular at an angle in the range from 30° to 60°, more preferably in the rage from 40° to 50° and most preferred at an angle of 45°.

At least one of the inlet or the outlet, may comprise an accommodation area for a bubble detection device, in particular a circular hollow hose having a smaller outer diameter than an end section of the inlet or outlet and/or being of the same material as the end section of the inlet or the outlet, in particular "einstückig", i.e. made as one piece. The accommodation area may be flexible to be squeezed in a channel of a sensor unit, in particular the bubble detection device, so that the surface of the accommodation area abuts against the measuring surface of the sensor unit. The sensor unit may be an ultrasound sensor and may be adapted to determine the density of the fluid flowing through a channel of the tubing connector in the accommodation area.

The membrane of the first pressure measurement chamber may be recessed with respect to an outer side of the pressure measurement chamber.

In an embodiment, the membrane of the first pressure measurement chamber may be flush, in part or completely, with respect to the outer side of the pressure measurement chamber.

In an embodiment, the membrane of the first pressure measurement chamber is not fixed on the outer side of the pressure measurement chamber.

"Outer side" may have the meaning of a side of the measurement chamber that is directed towards the measuring surface of the respective pressure measuring unit or may have the meaning of a wall surrounding the membrane. The membrane may be fixed and/or positioned inside a chamber formed by the walls forming the pressure measurement chamber.

Such a recessed configuration may allow for the membrane being better protected when the tubing connector is handled, e.g. laid down on a surface with the membrane being oriented towards the surface.

The first pressure measurement chamber may comprise a fixing element for fixing the first membrane to the tubing connector. In an alternative embodiment, the membrane may be fixed to the first pressure measurement chamber without a fixing element.

Preferably, the fixing element has a flat surface.

In an embodiment, the fixing element is recessed with respect to the pressure measurement chamber wall. Preferably, the fixing element is recessed, and the membrane is flush with the recessed fixing element.

The fixing element and/or the first membrane may be fixed to the tubing connector, in particular to a wall forming the pressure measurement chamber, by form-fit and/or force-fit and/or material-fit connection, in particular by ultrasonic welding, laser welding, heat welding, snap-fit, gluing or beading.

The first membrane may be fixed to the fixing element by form-fit and/or force-fit and/or material-fit connection, in particular by ultrasonic welding, laser welding, heat welding, snap-fit, gluing or beading.

The first pressure measurement chamber may comprise a circumferential edge, in particular a circular ring, over which the first membrane is stretched.

The first membrane may be formed as a separate part of the first pressure measuring chamber or the first membrane may form an integral part of the first pressure measuring chamber. "Forming part" has the meaning that the wall through which the pressure measuring chamber is defined corresponds to the pressure measuring chamber.

In accordance with a further embodiment at least one of the inlet, the outlet, the first tubing connector element and the second tubing connector element comprises an opening which is located in a side wall of the first pressure measuring chamber, wherein preferably the distance of the opening to the bottom of the first pressure measurement chamber is smaller than the distance of the opening to the membrane, wherein preferably the distance of the opening to the first membrane is maximal.

The pressure measuring chamber preferably has a first opening which fluidly communicates with the inlet or with the outlet and a second opening which fluidly communicates with the first or second tubing connector element wherein preferably said first and second openings are arranged in the same distance or in different distances from the membrane.

The at least one of the inlet, the outlet, the first tubing connector element and the second tubing connector element may be arranged with relation to the membrane so that the first membrane is not directly exposed to flow of liquid which enters the first pressure measurement chamber.

In an embodiment, the first pressure measurement chamber comprises a deflector that prevents direct flow of blood to the first membrane.

The first pressure measurement chamber may comprise one or more protrusion, such as domes and ribs, which extends from the inside wall, in particular the bottom of the chamber, preferably perpendicularly from the bottom of the chamber. "bottom" refers to a side of the pressure measurement chamber being opposite to the membrane of the pressure measurement chamber.

Said on or more ribs preferably extend radially or tangentially from said protrusion.

Preferably, the pressure measurement chamber comprises one or more stiffening elements, in particular cross-bars at the outside of the pressure measurement chamber in particular in a direction away from the membrane.

In accordance with an embodiment of the present invention the connector comprises said first and the second pressure measurement chamber, said second chamber comprising a second flexible membrane, also called measuring surface in this description Tthe first pressure measurement chamber may be directly fluidly connected to the inlet and the first tubing connector element and the the second pressure measurement chamber may be directly fluidly connected the outlet and the second tubing connector element. Alternatively, the first pressure measurement chamber may be directly fluidly connected to the outlet and the second tubing connector element and the second pressure measurement chamber may be directly fluidly connected to the inlet and the first tubing connector element.

"Directly fluidly connected" may have the meaning that there is channel leading directly into the respective chamber without a tubing in between.

It is emphasized that preferably the second pressure measurement chamber and/or the second membrane is configured according to any one of the above features which relate to the first pressure measurement chamber and/or the first membrane.

The membranes of the first and second pressure measurement chambers are preferably arranged in the same plane, in parallel planes or in planes which are arranged at an angle with respect to each other.

In particular arranging the membranes of the first and second pressure measurement chambers in the same plane may have the advantage that the coupling to the first and second pressure measuring units may be simplified as the measuring surfaces of the first and second pressure measuring units may also be provided in common plane and the movement of the measuring surfaces may be enabled by a common mechanics as the measuring surfaces have to travel the same distance for the coupling. Further, the space and size required for the tubing connector and the first and second pressure measuring units may be minimized.

The first and second membranes may be formed by separate membranes and/or the first and second membranes may have identical dimensions and in particular may be identical.

When the tubing connector is in a position with the plane of the membranes being vertical and at least one of the first and second longitudinal axes extending horizontally, the first chamber and the second chamber may be located at the same vertical position or are offset from each other in the vertical direction.

The first and second pressure measurement chamber may have an identical or different internal structure.

The Tubing connector according to any embodiment may further provide a structure in which the longitudinal axis of at least two of the inlet, the outlet, the first tubing connector element and the second tubing connector element are arranged in a first plane and wherein the membrane(s) is/are arranged in the same plane or in a second plane which is parallel to the first plane or which is at an angle with respect to the first plane. Said angle may be in the range between 75° and 105°, preferably between 85° and 95° and most preferred 90°. It is noted that the term "first plane" in which at least two of the inlet, the outlet, the first tubing connector and the second tubing connector lie encompasses the case that this first plane is actually a plane as well as the case that such plane is inclined or twisted due to the fact that at least two of the inlet, the outlet, the first tubing connector and the second tubing connector are not located at the same level. In the latter case the "frist plane" is a projected plane of at least two of the inlet, the outlet, the first tubing connector and the second tubing connector.

The first longitudinal axis and the second longitudinal axis may lie on one common straight line or on lines which are parallel to each other or form an angle with each other which is in the range between 75° and 105°, preferably between 85° and 95° and most preferred 90° and / or the third longitudinal axes and fourth longitudinal axis may form an angle with each other which is in the range between 75° and 105°, preferably between 85° and 95° and most preferred 90°.

In accordance with an embodiment a pump-tubing is connected to the first tubing connector element and the second tubing connector element and/or a first tubing is connected to the inlet and a second tubing is connected to the outlet, wherein the said tubing may be an extracorporeal tubing adapted to be used in a dialysis machine. The first tubing may be a section of the arterial tubing which is in during treatment connected to a patient and the second tubing may be a section of the arterial tubing which is during treatment connected to the filter.

The first tubing may comprise a first branch tubing and the second tubing may comprise a second branch tubing, wherein the first branch tubing and the second branch tubing may be connected to each other via a connection portion.

Only a single pump-tubing may be connected to the tubing connector.. There may be no further pressure measuring surfaces or membranes being provided in the tubing connector than the membranes of the first and second pressure measurement chambers. The tubing connector may be provided without any one or more than one or all of the following building elements within its formstable body: a check valve, a clot catcher, a phantom valve, a single needle chamber, a suction port for generating a vacuum between the tubing connector and a front surface of the blood treatment device.

In the figure section below embodiments of the inventions are described. Even though features are described in combination, not all must be present in every embodiment of the inventions. Any feature as described may be considered an independent feature.

Identical parts and parts with identical function are annotated with identical reference numerals in die figures.
Figure 1 shows two different embodiments of the arrangement comprising a pump, pressure measuring unit, tubing connector and pump-tubing in different views,
Figure 2 I is a front view on a part of a dialysis machine with the arrangement of figure 1 in one embodiment, Figure 2 II is a front view of a dialysis machine with the arrangement of figure 1 in one embodiment,
Figure 3 is a perspective view of one embodiment of the arrangement comprising a pump, pressure measuring unit, tubing connector and pump-tubing with detailed figures,
Figure 4 is a back and front view of the tubing connector in one embodiment,
Figure 5 is a front view of the tubing connector of figure 4 without membranes,
Figure 6 is a is a side view and a sectional view through the connector of figure 4,
Figure 7 is a detail of the embodiment of figure 6,
Figure 8 is a perspective and a front view of the tubing connector in a further embodiment,
Figure 9 is a top view of the connectors of both embodiments with the pump-tubing an in part with the extracorporeal tubing connected to the tubing connector,
Figure 10 are different views of a part of a dialysis machine with the arrangement of figure 1 in one embodiment, the tubing connector and a detail thereof in one embodiment and a sectional view of the arrangement including a tubing connector and the pressure measurement unit,
Figure 11 shows a concept with two pressure measurement units an embodiment for positioning the tubing connector,
Figure 12 shows a perspective view of the tubing connector according to a further embodiment,
Figur 13 shows a top view of a tubing connector according to a further embodiment with an inlet and an outlet which are arranged on the same straight line and
Figure 14 shows a view of the tubing connector of figure 13 from the side of the membranes of the pressure measurement chambers,
Figures 15 -19 show an embodiment of the invention with a guide plate in the pressure measurement chamber.

The pump and pressure measuring arrangement 1000 comprises a roller pump R, which is a peristaltic pump, and which is surrounded in part by the pump bed PB. The pump comprises a stator being connected to a motor for driving a rotator to which two (or more) rollers of the roller pump are attached. In pumping operation, the pump-tubing T is positioned in the pump bed PB and the rollers when running over the pump-tubing T squeeze the tube pushing a liquid inside the tube forward.

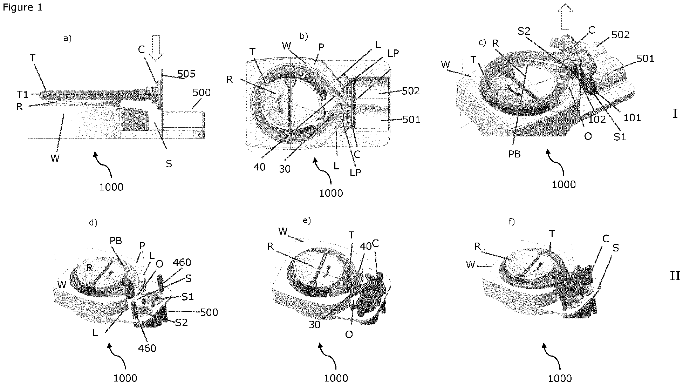

The pump bed PB comprises a wall W the shape of which is basically defined by the requirements of the peristaltic pump action. In the embodiment shown in figure 1 the pump bed PB forms at least a part of a circle. Further, there is provided one open part or opening O in the pump bed wall W through which the pump-tubing T runs when the pump-tubing T is arranged in the pump bed PB. In the area of the open space O the wall W protrudes in form of a protrusion P from a circular pump bed wall course.

Thus, the protrusion P comprises an open part O which is located between the pump bed PB and the accommodation space S, wherein the open part O defines a space through which the pump-tubing T runs when connected to the arrangement 1000.

The protrusion P comprises two, preferably symmetrical legs L, extending from the circular pump bed wall W in the direction of the accommodation space S.

The legs L have two portions LP where in the embodiment of figure 1 I a tubing connector C abuts in an operation condition in a direction along the normal of a plane of a measuring surface S1, S2 and in which a pressure measuring unit 500 is in contact with the tubing connector C. In the embodiment of figure 1 II the tubing connector C may abut in an operation condition in a direction perpendicular to the normal of a plane of the measuring surface.

Those portions LP face the accommodation space S in which the tubing connector is located C in the operation condition.

Figure 1a-1c show the pump and pressure measuring arrangement 1000 with the tubing connector C and the pump-tubing T before (figure 1a) and after (figure 1c) arranging the pump-tubing T in the pump bed PB and arranging the tubing connector C in the accommodation space S.

Figure 1a depicts a side view, figure 1b a top view and figure 1c a perspective view.

The direction in which the pump-tubing T is arranged in pump bed PB and the tubing connector C is arranged in the accommodation space S is from top to the bottom according to the arrow in figure 1a. The direction in which the pump-tubing T is removed from pump bed PB and the tubing connector C is removed from the accommodation space S is from the bottom to the top according to the arrow in figure 1c.

The accommodation space S is located between the end portion LP of the legs L and a measuring plane 505 of the a pressure measuring unit 500 of the pump and pressure measuring arrangement 1000.

The pressure measuring unit 500 may consist of two first and second pressure measurement units 501 and 502, which are located in parallel and have the same external shape, in particular the same external and internal structure. Each of the pressure measuring units 501 and 502 has a measuring surface S1, S2. It is noted that also for the embodiment shown in figure 1 II two corresponding measuring units of the measuring unit 500 are present even not separately depicted but indicated by their measuring surfaces S1 and S2.

A part of the first and of the second pressure measuring unit 501, 502 is moveable from and towards the accommodation space S, wherein the front face of the movable part of the first and second pressure measuring unit 501, 502 forms the measuring surface plane 505 defined by the plane of the measuring surfaces S1, S2 of said pressure measuring units 501,502.

In the embodiment of figures 1a to 1c the measuring surface plane 505 runs in a vertical direction relative to the bottom of the accommodation space S.

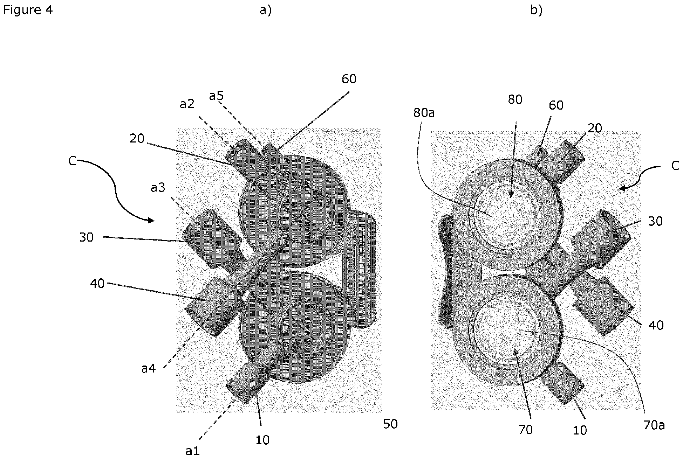

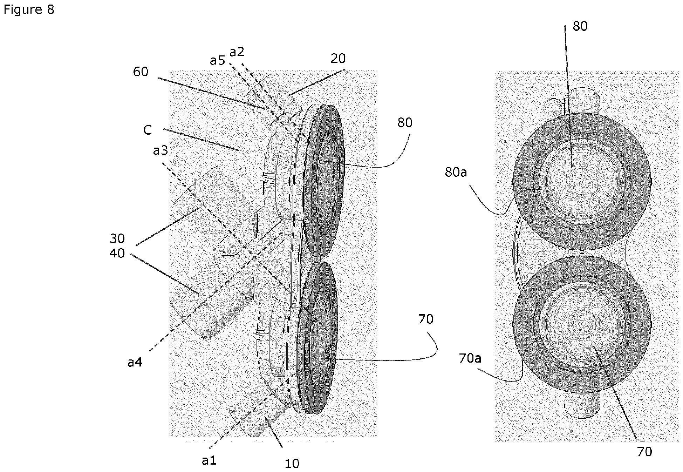

The tubing connector C is provided with a first and a second pressure measurement chamber which are a pre and a post pump pressure measurement chamber 70 and 80 as shown in more detail in figures 4 and 8. "pre pump" has the meaning that in normal operation of the pump R the pressure measurement chamber 70 is located upstream of the pump R and "post pump" has the meaning that in normal operation of the pump R the pressure measurement chamber 80 is located downstream of the pump R. This does not exclude that the pump may also be operated in the opposite direction, thereby reversing the flow. The crucial aspect is that the pressure measuring chambers 70 and 80 are not both downstream or upstream of the flow with respect to the the pump R, but one of the two is always upstream and the second one is always downstream with respect to the the pump R. The pressure measurement chambers 70 and 80 each have a membrane which cover this side of the pressure measurement chambers 70, 80 at least partially or completely which faces the measuring surface plane 505 of the pressure measuring units 501, 502.

After inserting the tubing connector C into the accommodation space S and after locating the pump-tubing T in the pump bed PB the a part of pressure measuring unit 500 is moved in a direction of the pressure measurement chambers 70 and 80 of the tubing connector C in order to perform a coupling process between the pressure measuring unit 500 and the membranes of the pressure measurement chambers of the tubing connector C. In other words, the pressure measuring unit 500 has a part that is fixed in its position relative to the pump R and/or the tubing connector C and another part that is movable towards the pump R and/or the tubing connector C. In particular, the movable part may comprise the measuring surfaces S1 and S2.

The accommodation space S is designed in order to allow for an automatic coupling of the membranes of the measuring chambers 70 and 80 of the tubing connector C to the measuring surfaces S1, S2 of the pressure measuring unit 500. At the same time the accommodation space S allows for positioning of the tubing connector C in the accommodation space S preferably without physical interaction with the measuring surfaces S1, S2 of the pressure measuring unit 500 and the measuring surfaces S1, S2 of the pressure measuring unit 500 may preferably not experience any friction or deformation upon positioning of the tubing connector C.

The accommodation space S is designed such that the tubing connector T fits into the space, wherein fitting has the meaning that substantially no other component may be positioned in addition in the accommodation space S. This may be described that after accommodating the tubing connector C in the accommodation space S there is only a slit or no slit at all present between the portions LP and the tubing connector C and the pressure measuring unit 500 and the tubing connector C.

As further shown in figures 1a to 1c, 1e and 1f the pump-tubing T is fluidly connected to the tubing connector C. This connection is made by use of a first tubing connector element 30 and a second tubing connector element 40 of the tubing connector C which are shown and explained in more detail below.

The pump-tubing T is arranged in a plane T1 which is perpendicular to the pressure measuring surface 505 in the embodiment of figures 1a to 1c. It is noted and as it is apparent from figure 1, 3, 4, and 8 the tubing connector elements 30, 40 may be located at slightly different heights w.r.t e.g. the pump bed PB or a plane perpendicular to the measuring surfaces S1, S2. This has the consequence that the plane T of the pump-tubing T may be considered slightly inclined, however, this slight incline shall still not being considered deviating from the perpendicular orientation of the plane T1.

Further, figure 1c shows a ring 101, 102 of each of the pressure measuring units 501 and 502. These rings 101, 102 may abut or may be in distance through a slit with corresponding rings of the pressure measurement chambers 70, 80 when the tubing connector C is its operating condition.

Figures 1d to 1f show an alternative embodiment. In this embodiment the measuring surfaces S1 and S2 of the pressure measuring unit 500, more precisely the first and second pressure measuring unit 501 and 502, are located in a plane which is parallel to the plane T1 in which the pump tubing T extends and /or which is parallel to surface of the accommodation space S. Again, in this embodiment the membranes of the pressure measuring units 501, 502 are in a plane which is parallel to the plane of the measuring surfaces S1, S2. As noted above, the reference numbers 501 and 502 are omitted in the figures 1d to 1f for illustration understanding purpose only.

Compared to the embodiment of figures 1a to 1c the pressure measuring unit 500 is turned by 90° Accordingly, the design of the tubing-connectors C differs from the design of the tubing connector shown in figures 1d to 1f. In the embodiment of figures 1a to 1c the longitudinal axes of the first 30 and second tubing connector elements 40 of the tubing connector C intersect the measuring surface plane 505 and/or further the plane in which the membranes of the first and second pressure measuring chambers 70 and 80 are located.

In contrast thereto in the embodiment of figures 1d to 1f the longitudinal axes of the first 30 and second tubing connector elements 40 of the tubing connector C do not intersect the measuring surfaces S1, S2 and/or further do not intersect the plane in which the membranes of the first and second pressure measuring chambers 70 and 80 are located but run parallel to those surfaces / plane.

In the embodiment of figures 1 a to 1c the tubing connector C is fixed between the outer surface of legs L of the protrusion P of the pump bed PB and the pressure measuring unit 500.

In the embodiment of figures 1d to 1f in order to fix the tubing connector C in place when inserted in its operating condition the accommodation space S is provided with pins 460 which extend vertically from the surface of the accommodation space S.

In the operation condition the pins 460 extend into through holes of the tubing connector C.

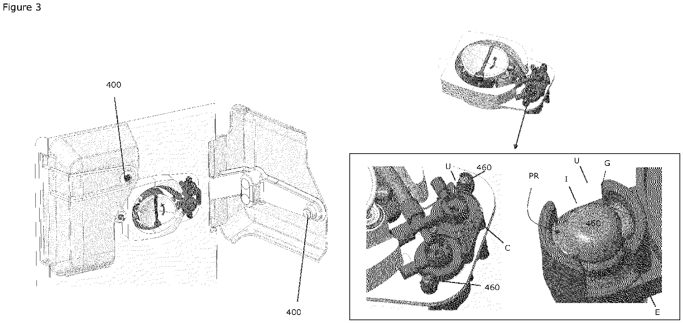

As more detailed shown in figure 3 the pins 460 are rod-shaped and have a head part with two projections PR. The projections PR fit into corresponding indentations I in a ring shaped contour of the connector C, which are shown in the detail according to figure 3. The connection between the tubing connector C and the head parts of the pins 460 may be a snap-fit connection. Alternatively or additionally the fixation can be achieved by rotating the pins 460 so that the projections PR interact with grooves G of the contour of the connector which is shown in figure 3. Alternatively or additionally the fixation may be achieved by a bayonet type connection where the pins 460 are rotated above an extension E of the tubing connector C

In the operating condition the measuring surfaces S1, S2of the pressure measuring unit 500 contact the membranes of the tubing connector C so that the movement of the membranes as a function of the pressure inside the pressure measurement chambers is transferred to the measuring surfaces S1 and S2. This transfer may then be detected by the respective pressure measuring unit 501, 502 and, thus, the measurement of the pressure of liquid, preferably blood, in the pressure measuring chambers of the tubing connector C can be carried out by the pressure measuring unit 500.

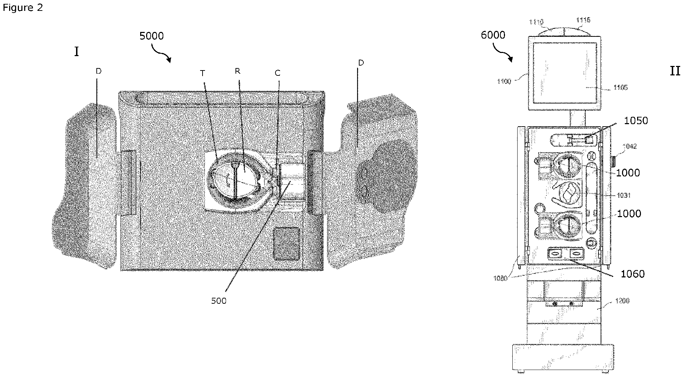

Figure 2 I show a view onto the arrangement of figure 1 together with a front part 5000 of first embodiment of a dialysis machine. The dialysis machine as an example for an extracorporeal blood treatment device, may comprise a door D. The door D comprises two parts which are swiveling by means of hinges located opposite each other. The use of a single-part door is also conceivable and covered by the present invention. Also the use of no door is also conceivable and covered by the present invention. When closed the door covers the arrangement of figure 1 at least in part, preferably in total. The door D or part of it may be configured to hold the tubing connector C in place in its operation condition.

Figure 2 II show a front view onto the arrangement of figure 1, in particular of figure 1 a) to c), together with a second embodiment of a dialysis machine 6000. The dialysis machine 6000 is an example for an extracorporeal blood treatment device. In this embodiment the pump and pressure measuring unit 1000 is provided as a blood pump. There may be more than one pump and pressure measuring units 1000 provided, for example it may in addition be provided as a substituate fluid pump for hemodiafiltration treatment. The dialysis machine 6000 uses a the tubing set and an dialyzer, which are not shown in order to simplify illustration, which are attached to the various components on the face of the machine 6000 prior to beginning a patient treatment. For example, the pump-tubing is attached to blood pump 1000 (depicted with the lower number 1000) and, depending on treatment mode and tubing set configuration, substituate pump 1000 (depicted with the lower number 1000). A dialyzer holder 1042 is provided for mounting the dialyzer, which is not shown. The dialysis machine 6000 also includes a monitor 1100 and a base portion 1200 , which houses the hydraulics of the machine 6000 . The monitor 1100 of this example provides a touchscreen display 1105 for user interface and a pair of indicator lights 1110 and 1115 , which are disposed above the display 1105 .

Also, the dialysis device 6000 may be provided with doors but other embodiments may have no or doors differently designed or attached. A set of doors 1080 may be attached to the front of the dialysis device 6000. The dialysis device 6000 may comprise a heparin pump 1050. The heparin pump 1050 may be configured to receive a syringe and the syringe may be connectable to the heparin tubing which may be connected to the heparin port of the tubing connector C. The dialysis device 6000 may comprise a blood temperature monitor 1060. The blood temperature monitor may be configured to receive the arterial and/or venous tubing and may allow for determining the temperature inside the arterial and/or venous tubing, wherein in particular the arterial tubing may be connected to the inlet of the tubing connector C. Inside the housing and/or the monitor of the dialysis device 6000 electronics (not shown) may be provided. Such electronics may in particular comprise one or more processors for controlling the operation of the dialysis device 6000. In particular the one or more processors may be configured to control the operation of the pump and pressure measuring unit 1000 and/or pump and pressure measuring units 1000.

It is noted that in other embodiments the arrangement 1000 of figure 1 d) to f) may be provided likewise in an extracorporeal blood treatment device as described by referring to figure 2.

Figure 4a shows a perspective view of the tubing connector C from the side which is not in contact with the pressure measuring unit 500 when the tubing connector C is in the operating condition and figure 4b shows a perspective view of the tubing connector C from its other side which is in contact with the pressure measuring unit 500 when the tubing connector C is its operating condition.

The tubing connector C comprises an inlet 10 (first system tube seat) which is fluidly connected to the arterial part, if the arrangement is used as blood pump, of the extracorporeal circuit and an outlet 20 (second system tube seat) which is fluidly connected via a dialyzer or any other filter to the venous part, if the arrangement used as blood pump, of the extracorporeal circuit. The inlet 10 comprises a first longitudinal axis a1, the outlet 20 comprises a second longitudinal axis a2. Reference numerals 30 and 40 relate to tube seats wherein 30 is the first tubing connector element having a third longitudinal axis a3 and 40 is the second tubing connector element having a fourth longitudinal axis a4. The first 30 and second tubing connector element 40 are fluidly connected to the ends of the pump-tubing T.

As may be seen from the drawings the denotation of the ports as inlet or outlet or in general the flowing direction through the tubing connector C is not a result of a mechanical or any other feature allowing a flow only in one direction. Thus, in use the inlet may be an outlet and vice versa. This also corresponds to the description provided above that the pump may run in both directions.

Figure 4b shows the same connector as shown in figure 4a from the other side. Reference numbers 70a and 80a each relate to membranes which are flexible and which cover the pre- and the post pump pressure measurement chambers 70 and 80, respectively. 70 denominates the pre pump pressure measurement chamber and 80 denominates the post pump pressure measurement chamber. In other word, when pump P is in operation blood or any other liquid flows first through chamber 70 into the pump-tubing T and then from pump-tubing T into chamber 80, which in terms of the flow direction is the downstream chamber while 70 forms the upstream chamber.

As can be seen from figure 4 the first 30 and second tubing connector elements 40 and their longitudinal axes a3 and a4 and thus connector elements for the roller-pump-tubing T run across each other. The same applies for the longitudinal axes a1 and a2 of the blood inlet 10 and blood outlet 20 which are connected in use to the extracorporeal circuit as outlined above. The tubing connector C may comprise a further connector element 60, which may be a heparin connector element for connecting a heparin source. In addition, the channels, which may include each a channel section with a smaller diameter than a section for accommodating the tubing, forming the first 30 and the second tubing connector 40 may cross each other.

The longitudinal axis of the further connector element 60 is denominated with a5.

The first 30 and second tubing connector elements 40 can be suitable for pump tubes with an inner and outer diameter 6,4 mm x 10 mm, but they can be easily adjusted to other dimensions of the pump tube, so that said dimensions are not limiting.

In the embodiment shown in figure 4, first longitudinal axis a1 is parallel to fourth longitudinal axis a4 and third longitudinal axis a4 is parallel to second longitudinal axis a2 and fifth longitudinal axis a5. The reason for this construction is for simplifying the mold construction. Anyway, other angles are possible and encompassed by the present invention.

The angle between the longitudinal axes a3 and a4 of the first and second tubing connectors 30 and 40 and/or the longitudinal axes a1 and a2 of the flow inlet 10 and flow outlet 20 may be 90°, or preferably in the range of 30° to 150°, however other angles are encompassed by the present invention as well. In particular, the longitudinal axes a3 and a4 of the first and second tubing connectors 30 and 40 may be 90° and the longitudinal axes a1 and a2 of the flow inlet 10 and flow outlet 20 may be larger or smaller than 90°.

In general, any angles that are described here refer to the smaller angle between the respective lines and/or planes.

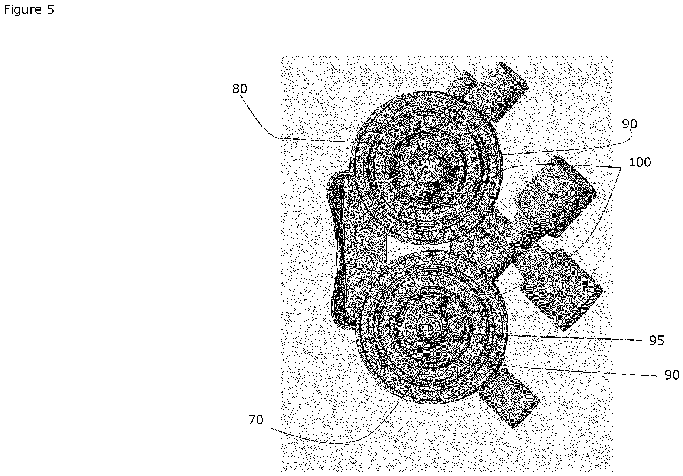

Figure 5 is a view according to the perspective of figure 4b, however without the membranes 70a and 80a. As can be seen from figure 5, the pressure measurement chambers 70, 80 are each surrounded by a circular ring 90 which totally extends around the pressure measurement chambers 70, 80 and which assures the tightness with the respective membranes 70a and 80a. The term "pressure measurement chamber" means that the chamber is suitable for a pressure measurement by an external pressure sensor, namely by pressure sensors of the pressure measuring unit 500.

As can be seen from figure 5 the interior of both pressure measurement chambers 70 and 80 may differ from each other. In the centre part of both chambers 70, 80 a dome shaped protrusion D may be present. From this protrusion D a number of ribs 95 may extend in chamber 70. In both chambers 70, 80 the bottom surface may have different levels. These features provide that the occurrence of bubbles in the blood flowing through the chambers 70, 80 is avoided, in particular when the chamber 70 is used a blood inlet chamber and chamber 80 is used as blood outlet chamber.

Furthermore, the tubing connector C may comprise a surface 100 for each of chambers 70. 80 which may be configured for ultrasonic welding. The surface 100 in this embodiment is located between two concentrically surrounding ribs, however, may be also made in any other form.

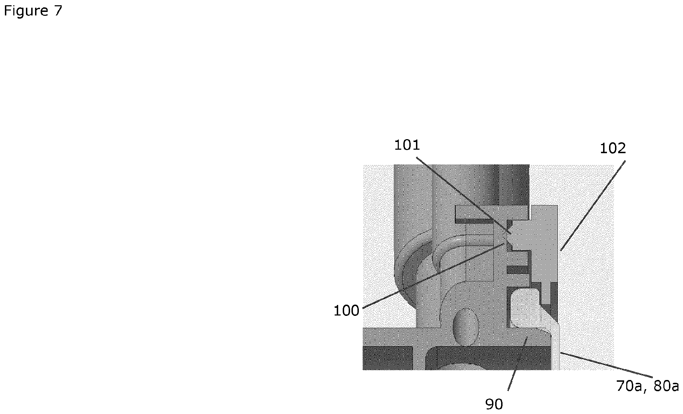

In the embodiment shown in figure 5 it is possible to fix the flexible membrane 70a, 80a through a circular element 102, not shown in figure 5 but in figure 7, which is fixed by ultrasonic welding to the surface 100 of the main body of the connector. Instead of being ultrasonic welded the circular element 102 may be fixed by snap or gluing or in any other suitable manner.

In another embodiment the membrane may be fixed by beading. In this case no circular elements 102 for fixing the membranes are needed.

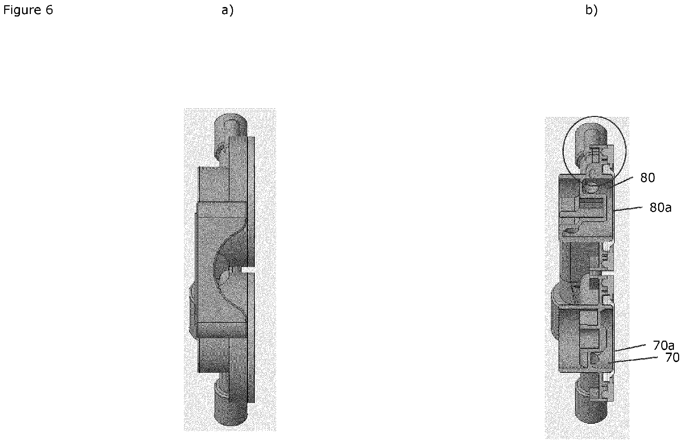

Figure 6a is a side view and figure 6b is a sectional view through the connector of figure 5.

Figure 7 is a view of the detail according to the encircled area in figure 6b. In figure 7 100 represents the surface area for ultrasonic welding and 101 the ultrasonic welding point. 102 is said circular element for fixing the membrane 70a, 80a. As shown in figure 4 the membrane is stretched over the circular rib 90 which forms an edge und the thickened margin area of the membrane 70a, 80a is overlapped in part by the circular ring member which serves as a fixing element 102 and which is securely fixed to the main body of the connector in area 101.

The fixing element 102 and/or the membrane 70a, 80a may be fixed to the tubing connector C, in particular to a wall forming the pressure measurement chamber 70, 80, by form-fit and/or force-fit and/or material-fit connection, in particular by ultrasonic welding, laser welding, heat welding, snap-fit, gluing or beading.

The membrane 70a, 80a may be fixed to the fixing element 102 by form-fit and/or force-fit and/or material-fit connection, in particular by ultrasonic welding, laser welding, heat welding, snap-fit, gluing or beading.

The pressure measurement chambers 70, 80 each comprise a circumferential edge , in particular a circular ring or rib 90, over which the membrane 70a, 80a is stretched as seen for example in figure 7.

In the embodiments of figures 4to 6 the membranes 70a, 80a are located in a plane which is parallel to the plane T1 of the pump-tubing T which interacts with the roller pump Ras for example shown in figures 1e and 1f.

Figure 8 depicts an alternative embodiment in which the pressure measurement chambers 70, 80 and thus the membranes 70a, 80a thereof are located perpendicularly to the plane T1 in which the pump-tubing T which interacts with the roller pump R is located. Thus, in the embodiment shown in figure 8 the pressure measurement chambers of the tubing connector C are rotated 90° compared to the previous embodiments of figures 4 to 6. The tubing connector C of figure 8 corresponds to the tubing connector which is shown in figures 1a to 1c.

The reference numerals 10, 20, 30, 40, 50, 70 and 80 in figure 8 denominate the same parts as explained in figure 4. As regards the orientation and angles of longitudinal axes a1, a2, a3, a4 and a5 relative to each other the same applies as in the embodiment of figure 4.

However, as seen in figure 8 all longitudinal axes a1 to a5 intersect the plane which is formed by the membranes 70a and 80a of the tubing connector C of figure 8.

While longitudinal axes a3 and a4 cross on one side of the membranes 70a, 80a the longitudinal axes a1 and a2 cross on the other side of the membranes 70a, 80a.

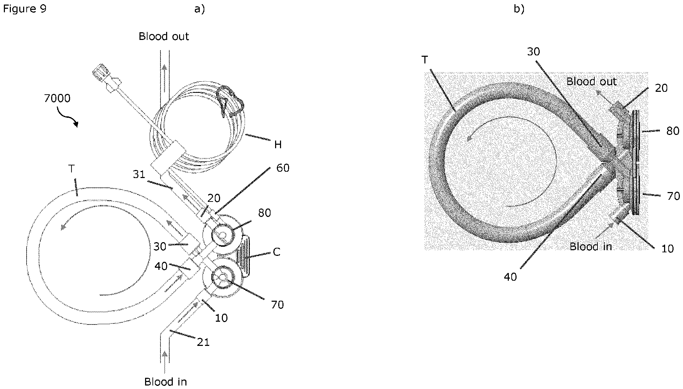

The longitudinal axes a1 and a2 of the inlet 10 and the outlet 20 lie in a first one plane. However, the longitudinal axes a3 and a4 are in a second one plane which becomes more evident from figure 9a. It is also referred to the description above that a slight offset as described when referring to pump-tubing plane is also part of the invention, i.e. does not mean that this slightly offset is understood as not being in one plane. The first one plane and the second one plane may be the same or different plane. The first one plane and the second parallel to each other.

In figure 9a a tubing set 7000 is schematically shown. The tubing set 7000 of this embodiment comprises a tubing connector C as described referring to figure 1 d) to f). In another embodiment the tubing set 7000 comprises s tubing connector C as described referring to figure 1 a) to c), other elements may be the same. The tubing set 7000 comprises a pump-tubing T, a tubing connector C, an arterial tubing having a first arterial tubing section 21 connected to the inlet 10 and a second arterial tubing section 31 connected to the inlet 10, and a heparin tubing H. The pump-tubing T which is configured to interact with the roller pump R is shown. The pump-tubing T runs loop-shaped. The exemplary direction of blood transport through the pump-tubing T including the rotation direction of the roller pump R is shown by arrows. Pump-tubing T is fluidly connected to the first and second tubing connector elements 30 and 40.. The arterial tubing section 31 may be configured to be connected to a filter or a dialyzer and/or the first arterial tubing section 21 may be configured to be connected to a needle or catheter for withdrawing blood from a patient e.g. may have a male or female luer connector at its end.

The first tubing connector element 30 and the fluid inlet are in fluid communication with the first pressure chamber 70. The second tubing connector 40 and the fluid outlet 20 are in fluid communication with the second pressure chamber 80. The tubing connector C shown in figure 9a corresponds to the tubing connector C described with reference to figure 4. The tubing connector C may also be the tubing connector C as described with reference to figure 8.

Thus, figure 9b shows the alternative embodiment in which the pressure chambers 70, 80 are rotated by 90° in relation to figure 9a so that the pressure chambers run vertically through the paper plane. The tubing connector shown in figure 9b corresponds to the tubing connector C shown in figure 8.

The tubing set 7000 may comprise one or more further components of the group of (not shown in the figures) a first branch line being connected to the second section 31, a venous line for guiding blood back to the patient being configured to be connected with one end to the dialyzer and to be connected to a needle or a catheter for returning blood to the patient with the second end e.g. may have a male or female luer connector at its end, a second branch line being connected to the venous line, wherein the first-branch line and the second branch line may be connected to each other via a T or Y piece.

The tubing set 7000 may be provided in one package with the venous line and the arterial line not being connected to each other and/or the first and the second branch line being connected to arterial line and the venous line respectively and/or the first branch line and the second branch line being connected to each other.

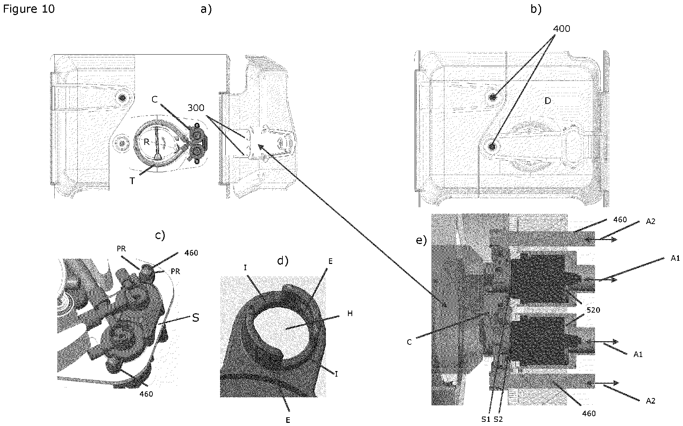

In figure 10c an enlarged view of the connector C according to an embodiment as describe referring to figure 4 is shown. Furthermore, as shown in figure 10c, the interface for connecting the connector to the body of the blood treatment device comprises two pins 460 for fixing the tubing connector C to the accommodation space S. The fixing elements, i.e. pins 460 are rod-shaped and have a head part with two projections PR, which are located on opposite sides of the rod which extends through hole H. The holes are located on end portions of the tubing connector C and the holes H and the centre portions of the chambers 70 and 80 may lie a straight line.

The projections PR fit into corresponding indentations I in a ring shaped contour of the connector C, which are shown in the detail according to figure 10d. The connection between the tubing connector C The fixation can be achieved by rotating the fixing elements 460 so that the projections PR interact with extensions E of the contour of the connector which is shown in figure 10d.

Said extensions E alternate in succession. When inserting the tubing connector C the projections PR of the pins 460 lie in the indentations I, after rotation of the pins 460 the projections PR interact with the extensions E so that the tubing connector C is fixed to the compression pins 460.

In figure 10e a sectional view of the arrangement of figure 10a is shown. The arrows A1 in figure 10e indicate that the pressure measuring surfaces S1 and S2 are moveable towards and/or away from the membranes 70a, 80a of the pressure measurement chambers 70, 80 of the tubing connector C, for example by means of a drive which may be a motor, one or more pneumatic cylinders 520. Alternatively or additionally the pins 460, indicated by arrows A2 may be configured and/or connected to drive for pulling towards and/or pushing away the tubing connector C from the measuring surfaces S1 and S2. In addition, the pins 460 may be rotatable by a drive fir fixing the connector. In an alternative embodiment the door D may push the connector C towards the surfaces S1 and S2. In this case the pins 460 and/or the surfaces S1 and S2 may be provided with the drives or not.

For inserting the tubing connector C onto the blood treatment device interface the user opens the port/door D in those embodiments in which a door D is present by pushing the buttons 400 which are shown in figure 10b. Then the connector is first positioned on the panel, i.e. into the accommodation space S (interface) and the pump-tubing T is located in the pump bed PB. Then the user closes the port by closing the doors D.

Once the port is closed, the two measuring surfaces S1 and S2 may move toward the membranes 70a, 80a for enabling a tight coupling between respective sensor and membrane. Alternatively or additionally the coupling my be achieved upon closing the door. Alternatively or additionally the coupling may be achieved by pulling the pins and, thereby the tubing connector C towards the surfaces S1 and S2. The door D may withstands the force generated for the coupling between surfaces S1, S1 and membranes 70a, 80a.

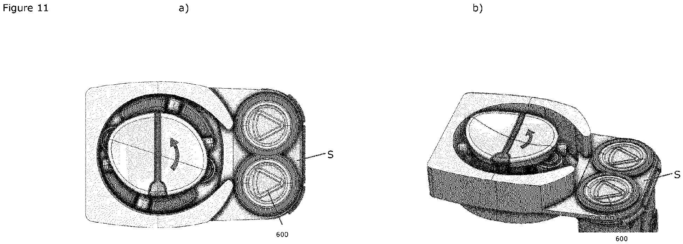

Figure 11 shows a concept with two pressure measurement units. The pressure measurement units comprise a cap 600. The cap may also be two caps, one for each of the pressure measuring units. Figure 10a) is a top view and figure 10b) a perspective view of the arrangement. It is noted that both figures show the pumping and pressure measuring arrangement without a tubing connector. The cap 600 may have a similar function as the pins described above. The cap 600 may be movable towards and away from the surfaces S1 and S2. Once the tubing connector is positioned in the accommodation space below the cap, the tubing connector may be movable with the caps. For positioning the tubing connector the cap 600 or the caps may be lids that can be opened. Alternatively, the cap 600 or the caps may have an opening on the side to that the tubing connector may be slided from the side under the cap 600 or caps.

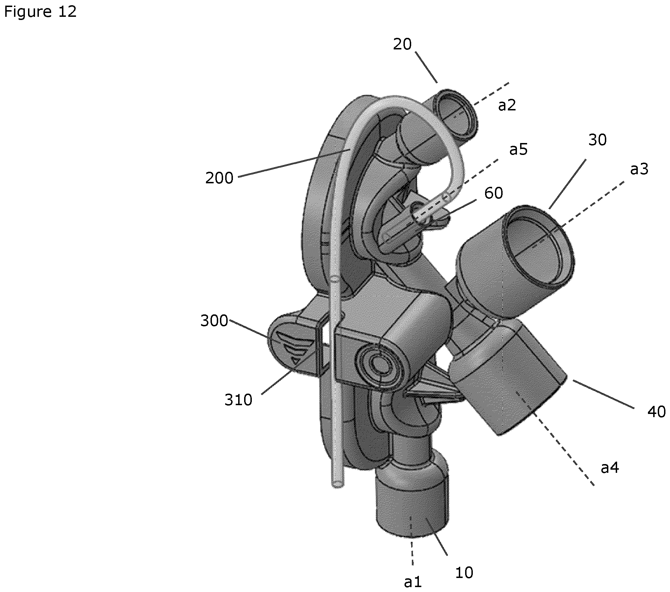

Figure 12 shows a tubing connector in accordance with a further embodiment.

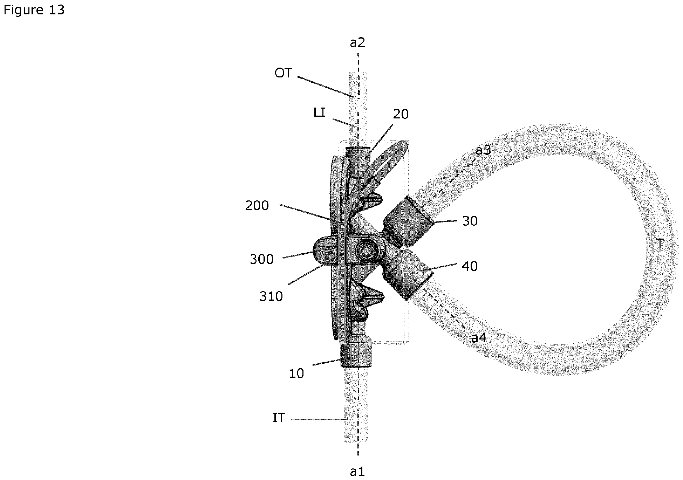

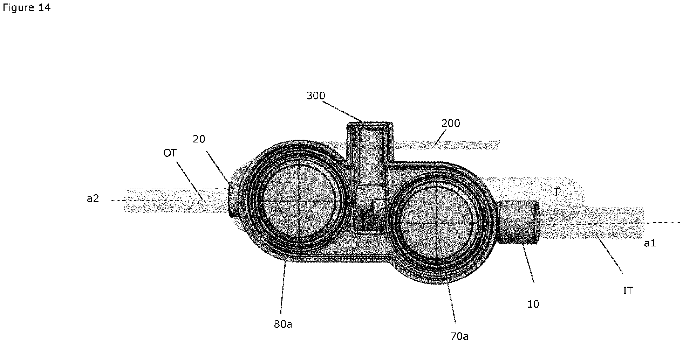

The inlet 10 comprises a first longitudinal axis a1, the outlet 20 comprises a second longitudinal axis a2. Reference numerals 30 and 40 relate to tube seats wherein 30 is the first tubing connector element having a third longitudinal axis a3 and 40 is the second tubing connector element having a fourth longitudinal axis a4. The first 30 and second tubing connector element 40 are fluidly connected to the ends of the pump-tubing T which is shown in figures 13 and 14.

In contrast to the embodiment of figure 8 the longitudinal axis a1 of the inlet 10 is parallel to the plane of the membranes of the pressure measurement chambers, while the longitudinal axis a2 of the outlet 20 has the same orientation as in figure 8 and intersects the plane of the membranes of the pressure measurement chambers of the tubing connector.

Figure 200 refers to a further tubing which is adapted to supply a fluid, such as heparine, through the further connector element 60 the longitudinal axis thereof is denominated with a5.

As shown in figure 12 the tubing 200 runs from the further connector element 60 to which it is tightly connected and makes then a U-turn. It then extends parallel to the axis a1 and is located and fixed in a groove 310 which is located at the top side of the tubing connector.

Reference numeral 300 denominates a push tab which is configured to be exerted to a pressure force of a user in order to push the tubing connector into its final position within the pump bed arrangement.

In the embodiment of figure 12 the angle between the axes a1 and a2 is in the range of 120 ° to 150° and preferably is 135°.

Figure 13 is a top view of a tubing connector with pump tubing T which is connected to the first tubing connector element 30 and to the second tubing connector element 40. Further blood inlet tubing IT is tightly connected with the inlet 10 and blood outlet tubing OT is tightly connected with the outlet 20.

In contrast to the embodiment of figure 12 both, the first and the second longitudinal axes of the first elongated channel of the inlet 10 and of the second elongated channel of the outlet 20 are located on the same straight line LI in the top view.

Apart from this all elements in figures 12 and 13 which have the same reference numerals are identical or at least identical in their function.

As can be clearly seen from figure 13, the first and second connector elements 30, 40 do not lie in the same plane, but are offset from each other in a direction perpendicular to the paper plane.

As a result, the tube T does not lie exactly in one plane, but in a slightly twisted plane. This is nevertheless considered a "plane" in the context of the present invention.

Figure 14 is a side view of the arrangement of figure 13 in a view from the left. Same reference numerals depict the same parts as in figure 13.

Figure 14 shows that the flexible membranes 70a and 80a of the pump measuring chambers of the tubing connector are not located on the same level. Instead membrane 80a is located above the membrane 70a when the connector is arranged in the position of figure 14 in which the longitudinal axes a1 and a2 run in a horizontal direction.

Further, figure 14 illustrates that the longitudinal axes a1 and a2 are offset in the vertical direction in the same amount as the membranes 70a and 80a. The same applies for the pressure measuring units which interact with the membranes when the tubing connector is in use.

While the longitudinal axes a1 and a2 lie on the same straight line LI in the top view as in figure 13 they run parallel and not on the same straight line in a side view according to figure 14.

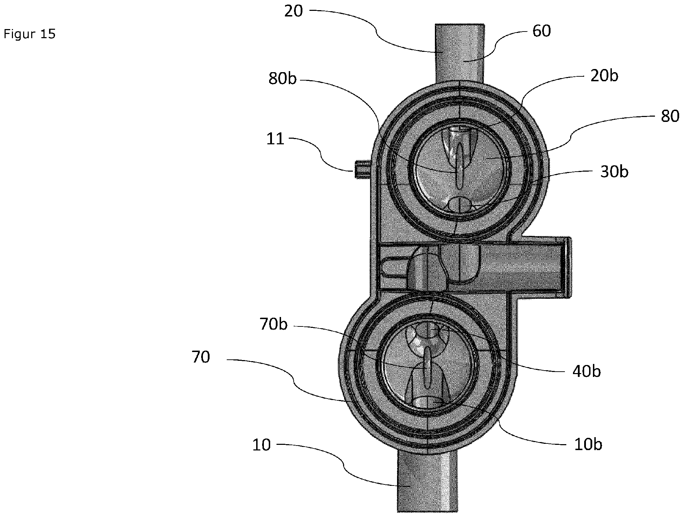

Figures 15 to 19 show an embodiment of the connector and its internal structure of the pressure domes 70 and 80.

This embodiment is a further embodiment of the connector according to Figures 13 to 14.

In Figures 15 to 19 same parts or parts having the same functionality as those in the embodiments in figures 1 to 14 are denominated with the same reference numerals.

Figure 15 shows the connector with the diaphragms 70a and 80a removed as viewed from the pressure sensors.

As shown in figure 15 the pressure measurement chambers 70 and 80 each comprise a guide plate or fin 70b or 80b, which protrude into the chambers and extend along the axes of the inlet 10 and the outlet 20.

The fins 70b and 80b are each located at the bottom of the respective measuring chambers 70, 80 and extend therefrom to the membrane (not shown in figure 15).

The end 10b of the inlet 10 and the beginning 40b of the pump connection 40 lead into the measuring chamber 70. The fin 70b extends between them, i.e. between the end 10b and the beginning 40b. The fin 70b is platelet-shaped and has a longitudinal dimension which is located on the imaginary line which connects the end 10b and the beginning 40b.

The end 30b of the pump connection 30 and the beginning 20b of the outlet connection 20 lead into the measuring chamber 80. The fin 80b extends between them. i.e. between the end 30b and the beginning 20b. The fin 70b is plateled-shpaed and has a longitudinal dimension which is located on the imaginary line which connects the end 30b and the beginning 20b.

The fins 70b and 80b protrude into the respective chambers without touching the membranes 70a or 80a during normal operation. If there is a pressure drop in the blood line, the two fins 70b and 80b prevent one or both membranes from being sucked into the respective pressure measurement chamber 70, 80 and blocking it.

As seen in figure 15 the fins 70b and 80b are not in direct contact with the beginnings 20b and 40b and ends 10b and 30b but are located in a distance therefrom.

The largest dimension of the fins 70b and 80b is smaller than the diameter of the pressure measurement chamber 70, 80 which is circular.

The fins 70b and 80b are located in the center portion of the bottoms of the pressure measurement chambers 70, 80. The fins 70b and 80b do not contact the circumferential border of the bottoms of the pressure measurement chambers 70, 80.

They are located on an imaginary line which intersects the bottoms of the pressure measurement chambers 70, 80 into two symmetrical parts.

The fins 70b, 80b in the pressure measurement chambers 70, 80 reduce the strain on the blood and thereby enhance the hemocompatibility. They minimize blood trauma and related risk factors, e.g., hemolysis (blood rupture), thrombosis (blood aggregation), and hemostasis (blood coagulation due to stagnation).

In addition, the fins 70b, 80b reduce the volume of the pressure measurement chambers and of the connector. This minimizes the blood volume in bloodline.

The fins 70b, 80b further ensure that the blood flowing into the pressure measurement chamber 70, 80 is evenly distributed in the chamber 70, 80, thereby improving the accuracy of the pressure measurement.

Each pressure measurement chamber 70, 80 may comprise exactly one or two or more fins. Preferably, only one fin 70b, 80b is fitted in each pressure measurement chamber 70, 80.

Figure 15 also shows a pin 11, which is used to interact with a sensor on the surface of the pump bed PB and indicates the presence of the connector C. After completion of a dialysis treatment, the connector C can be ejected with the pin 11.

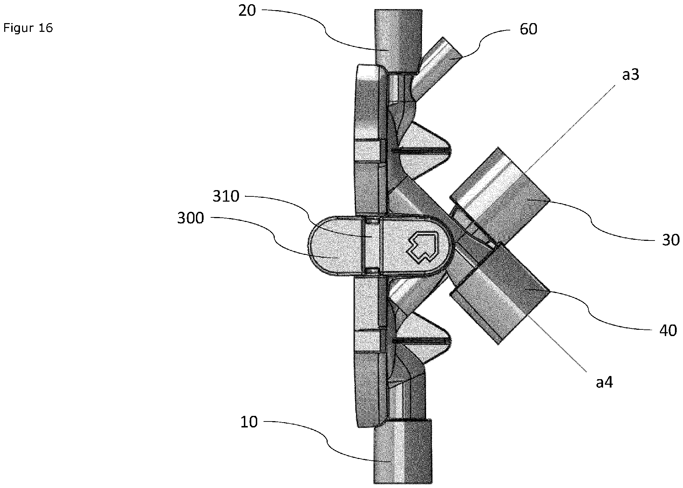

Figure 16 shows a top view of the connector as in Figure 13, but without installed pump tube T, supply line IT and discharge line OT as well as heparin line 200.

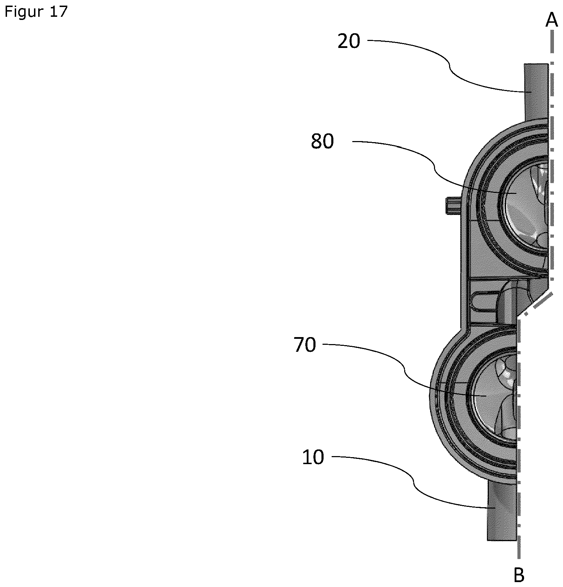

Figure 17 is a view on the bottom side of the connector according with figure 15, wherein in figure 17 the part of the connector which is located on the right hand side of line A-B is omitted.

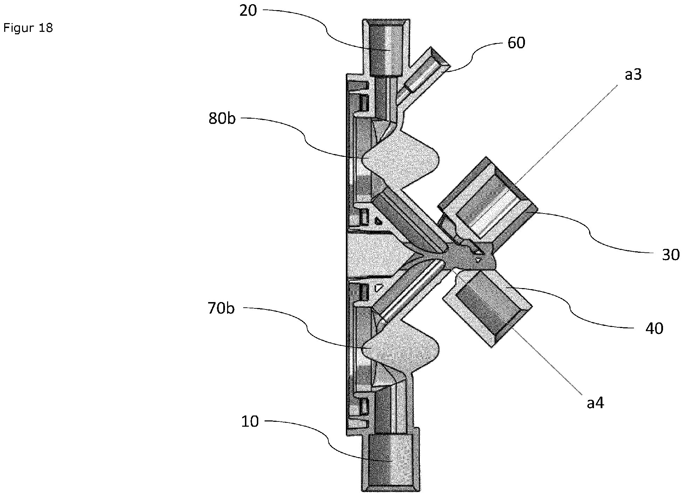

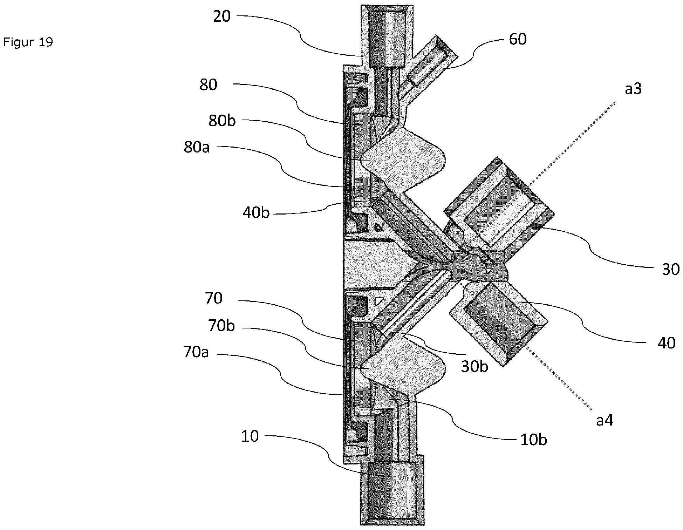

Figures 18 and 19 show a sectional view of the connector along the axis or cutting line A-B as shown in Figure 17.

Figure 18 shows the section through the connector along line A-B without membranes 70a and 80a.

In figure 18 fins 70b and 80b are shown in their longitudinal section. This figure shows that both fins 70b and 80b in their sectional view have the shape of a triangle with a rounded tip as regards the portion of the fins which extends into the pressure measurement chamber 70, 80. The fin is located in the chambers 70, 80 so that the rounded tip has the smallest distance to the membranes 70a and 80a compared to other portions of the fins 70b and 80b. Said membranes are shown in figure 19.

Figure 19 shows the section through the connector along line A-B in figure 17 with installed membranes 70a and 80a.

As further shown in figure 19 the end 10b of the inlet 10 and the beginning 40b of the pump connection 40 lead into the measuring chamber 70. The fin 70b extends between them. Further, the end 30b of the pump connection 30 and the beginning 20b of the outlet connection 20 lead into the measuring chamber 80. The fin 80b extends between them.

It is stressed that the arrangement of one or more fins in the pressure measurement chambers is not restricted to the embodiment of figures 15-19 but is also conceivable and part of this disclosure in case that the connector is one of the connectors which are depicted in figues 4 and 5. In the connectors of figures 4 and 5 the longitudinal axes a1 to a5 do not intersect the plane which is formed by the membranes 70a and 80a of the tubing connector C.

Further aspects of the invention refer to the following aspects and the aspects may be combined with the features as described above, aspects that may not be for technical reasons reasonably combined may not be combined, not limiting any embodiment described by under technical perspective combinable aspects:
Aspect 1: Tubing connector having an inlet for a liquid and an outlet for the liquid as well a first tubing connector element and a second tubing connector element, wherein the inlet and the outlet are both configured to be fluidly connected with a tubing and wherein the first and the second tubing connector elements are both configured to be fluidly connected to a peristaltic pump-tubing, wherein the tubing connector comprises at least one first pressure measurement chamber for measuring the pressure of the liquid which is present in said first chamber having a chamber wall with an opening for the pressure measurement, said first chamber comprising a first flexible membrane which in part or fully covers said opening wherein the first chamber is fluidly arranged between the inlet and the first tubing connector element or between the blood outlet and the second tubing connector element.
Aspect 2: Tubing connector according to any other aspect, wherein the inlet comprises a first elongated channel having a first longitudinal axis and the outlet comprises a second elongated channel having a second longitudinal axis, and/or wherein the first tubing connector element comprises a third elongated channel having a third longitudinal axis and the second tubing connector element comprises a fourth elongated channel having a fourth longitudinal axis.
Aspect 3: Tubing connector according to any other aspect, wherein the first longitudinal axis and the second longitudinal axis are not parallel to each other and/or the third longitudinal axis and the fourth longitudinal axis are not parallel to each other.
Aspect 4: Tubing connector according to any other aspect, wherein the first longitudinal axis and the second longitudinal axis form an angle with each other which is in the range between 75° and 105°, preferably between 85° and 95° and most preferred 90° and / or the third longitudinal axes and fourth longitudinal axis form an angle with each other which is in the range between 75° and 105°, preferably between 85° and 95° and most preferred 90°.
Aspect 5: Tubing connector according to any other aspect, wherein the third and fourth longitudinal axes are parallel to a plane of the membrane or wherein the third and fourth longitudinal axes intersect a plane of the membrane in one plane or each or in pairs in planes parallel to each other.
Aspect 6: Tubing connector according to any other aspect, wherein the first and fourth longitudinal axes are parallel to each other and/or wherein the second and third longitudinal axes are parallel to each other.
Aspect 7: Tubing connector according to any other aspect, wherein the tubing connector comprises a further fluid inlet having a channel with an opening into said first chamber.
Aspect 8: Tubing connector according to any other aspect, wherein a fifth longitudinal axis of the channel of the further fluid inlet is parallel to the second and third longitudinal axis, and preferably the fifth, the second and the third longitudinal axis lie in a same plane.
Aspect 9: Tubing connector according to any other aspect, wherein the fifth longitudinal axis intersects the plane of the membrane or is parallel to the plane of the membrane or is tangential to the first pressure measurement chamber, wherein the first pressure measurement chamber has a circular base.
Aspect 10: Tubing connector according to any other aspect, wherein the connector does not comprise any other connector but the inlet, the outlet, the first tubing connector element, the second tubing connector element and the further fluid connector.
Aspect 11: Tubing connector according to any other aspect, wherein the inlet and the first tubing connector element end in the pressure measurement chamber or the outlet and the second tubing connector element end in the pressure measurement chamber.
Aspect 12: Tubing connector according to any other aspect, wherein at least two of the first, the second, the third, and the fourth longitudinal axes form a plane, which plane is the same plane as that of the membrane or a parallel plane or which extends in an angle with respect to a plane of the membrane, in particular perpendicularly.
Aspect 13: Tubing connector according to any other aspect, wherein the longitudinal axis of at least one of the inlet, the outlet, the first tubing connector element and the second tubing connector extends in an angle with respect to a plane of the membrane, in particular in an angle in the range from 30° to 60°, more preferably in the rage from 40° to 50° and most preferred in an angle of 45°.
Aspect 14: Tubing connector according to any other aspect, wherein at least one of the inlet, the outlet, comprises an accommodation area for a bubble detection device, in particular a circular hollow hose having a smaller outer diameter that an end section of the inlet or outlet and/or being of the same material as the end section of the inlet or the outlet, in particular "einstückig", i.e. made of one piece.
Aspect 15: Tubing connector according to any other aspect, wherein the tubing connector comprises a first pressure measurement chamber and the first membrane is recessed with respect to an outer side of the pressure measurement chamber.
Aspect 16: Tubing connector according to any other aspect, wherein the first pressure measurement chamber comprises a fixing element for fixing the first membrane to the connector or wherein the membrane is fixed to the first chamber without a fixing element.
Aspect 17: Tubing connector according to any other aspect, wherein the fixing element has a flat surface.
Aspect 18: Tubing connector according to any other aspect, wherein the fixing element and/or the first membrane is fixed to the tubing connector by form-fit and/or force-fit and/or material-fit connection, in particular by ultrasonic welding, laser welding, heat welding, snap-fit, gluing or beading.
Aspect 19: Tubing connector according to any other aspect wherein the first pressure measurement chamber comprises a circumferential edge, in particular a circular ring, over which the first membrane is stretched.
Aspect 20. Tubing connector according to any other aspect, wherein the first membrane is formed as a separate part of the first chamber or wherein the first membrane forms an integral part of the first chamber.
Aspect 21 :Tubing connector according to any other aspect, wherein the at least one of the inlet, the outlet, the first tubing connector element and the second tubing connector element comprises an opening which is located in a side wall of the first chamber, wherein the distance of the opening to the bottom of the first pressure measurement chamber is smaller than the distance of the opening to the membrane, wherein preferably the distance of the opening to the first membrane is maximal.
Aspect 22: Tubing connector according to any other aspect, wherein the chamber has a first opening which fluidly communicates with the inlet or with the outlet and a second opening which fluidly communicates with the first or second tubing connector element, wherein said first and second openings are arranged in the same distance or in different distances from the membrane.
Aspect 23: Tubing connector according to any other aspect, wherein the at least one of the inlet, the outlet, the first tubing connector element and the second tubing connector element are arranged with relation to the membrane so that the first membrane is not directly exposed to flow of blood which enters the first pressure measurement chamber.
Aspect 24: Tubing connector according to any other aspect, wherein the first pressure measurement chamber comprises a deflector that prevents direct flow of blood to the first membrane.
Aspect 25: Tubing connector according to any one of the preceding claims, wherein the first pressure measurement chamber comprises one or more protrusion, such as domes and ribs, which extends from the inside bottom of the chamber, preferably perpendicularly from the bottom of the chamber.
Aspect 26: Tubing connector according to any other aspect, wherein on or more ribs extend radially or tangentially from said protrusion.
Aspect 27: Tubing connector according to any other aspect, wherein the pressure measurement chamber comprises one or more stiffening elements, in particular crossbars at the outside of the chamber.
Aspect 28: Tubing connector according to any other aspect, wherein the connector comprises said first and a second liquid pressure measurement chamber, said second chamber comprising a second flexible membrane, wherein the first chamber comprises the inlet and the first tubing connector element and the wherein the second chamber comprises the blood outlet and the second tubing connector element.
Aspect 29: Tubing connector according to any other aspect, wherein the second pressure measurement chamber and/or the second membrane is configured according to any one of claims 1 to 27.
Aspect 30: Tubing connector according to any other aspect, wherein the wherein the membranes of the first and second pressure measurement chambers are arranged in the same plane, in parallel planes or in planes which are arranged at an angle with respect to each other.
Aspect 31: Tubing connector according to any other aspect, wherein the first and second membranes are formed by separate membranes and/or wherein the first and second membranes have identical dimensions and in particular are identical.
Aspect 32: Tubing connector according to any other aspect, wherein the first and second pressure measurement chamber have an identical or different internal structures.
Aspect 33: Tubing connector according to any other aspect wherein the longitudinal axis of at least one of the inlet, the outlet, the first tubing connector element and the second tubing connector element are arranged in a first plane and wherein the membrane(s) is/are arranged in the same plane or in a second plane which is parallel to the first plane or which is in an angle with respect to the first plane.
Aspect 34: Tubing connector according to any other aspect, wherein the angle is in the range between 75° and 105°, preferably between 85° and 95° and most preferred 90°.
Aspect 35: Tubing connector arrangement comprising a tubing connector according to any other aspect, wherein a pump tubing is connected to the first tubing connector element and the second tubing connector element and/or a first tubing is connected to the inlet and a second tubing is connected to the outlet
Aspect 36: Tubing connector arrangement according to any other aspect, wherein the first tubing comprises a first branch tubing and the second tubing comprises a second branch tubing, wherein the first branch tubing and the second branch tubing are connected to each other via a connection portion and, wherein on the tubing connector arrangement only one pump-tubing is provided.
Aspect 37: Tubing connector arrangement according to any other aspect, wherein the tubing connector comprises a first pressure measurement chamber being fluidly connected to the inlet and the first tubing connector and a second pressure measurement chamber being fluidly connected to the outlet and the second tubing connector.
Aspect 38: Blood treatment device, comprising:
   a pump, preferably a roller pump for pumping a liquid, preferably blood,
   a tubing connector arrangement according to any other aspect,
   wherein the pump tubing fluidly connects the roller pump with the tubing connector,
   wherein the tubing is at least in part is in contact with the roller pump, so that when the roller pump is rotating a liquid is conveyed through the pump tubing.
Aspect 39: Blood treatment device according to any other aspect, wherein the tubing connector comprises a first and a second pressure measurement chamber, wherein the first chamber is located upstream of the second chamber in terms of the liquid flow through the tubing connector.

### List of Reference signs:

pump and pressure measuring arrangement 1000
roller pump R
pump bed PB
pump-tubing T
wall W
open part or opening O
protrusion P
accommodation space S
legs L
line LI
portions LP
tubing connector C
pressure measuring unit 500
tubing connector C
measuring plane 505
first pressure measurement units 501
second pressure measurement units 502
pre-blood pump pressure measurement chamber 70
post-blood pump pressure measurement chamber 80
rings 101, 102
measuring surfaces S1 and S2
plane, in which the pump tubing T extends T1
pins 460
front part 5000
door D
dialysis machine 6000
dialyzer holder 1042
a monitor 1100
base portion 1200
touchscreen display 1105
pair of indicator lights 1110 and 1115
set of doors 1080
heparin pump 1050
blood temperature monitor 1060
blood inlet 10
pin 11
blood outlet 20
first longitudinal axis a1
second longitudinal axis a2
first tubing connector element 30
third longitudinal axis a3
second tubing connector element 40
fourth longitudinal axis a4
ribs 95
flexible membrane 70a, 80a
fins 70b, 80b
circular element 102
circular rib 90
area 101
fixing element 102
circumferential edge 90
grooves G
indentations I
extension E
tubing set 7000
first arterial tubing section 21
first arterial tubing section 31
heparin tubing H
pneumatic cylinders 520
button 400
cap of the pressure measurement units 600

## Claims

1. Tubing connector having an inlet for a liquid and an outlet for the liquid as well a first tubing connector element and a second tubing connector element, wherein the inlet and the outlet are both configured to be fluidly connected with a tubing and wherein the first and the second tubing connector elements are both configured to be fluidly connected to a peristaltic pump-tubing,
wherein the tubing connector comprises at least one first pressure measurement chamber for measuring the pressure of the liquid which is present in said first chamber having a chamber wall with an opening for the pressure measurement, said first chamber comprising a first flexible membrane which in part or fully covers said opening,
wherein the first chamber is fluidly arranged between the inlet and the first tubing connector element or between the outlet and the second tubing connector element,
wherein the inlet comprises a first elongated channel having a first longitudinal axis and the outlet comprises a second elongated channel having a second longitudinal axis, and/or wherein the first tubing connector element comprises a third elongated channel having a third longitudinal axis and the second tubing connector element comprises a fourth elongated channel having a fourth longitudinal axis,
wherein at least one or two or three or all four longitudinal axes intersect a plane of the membrane at an angle between 75° and 105°, preferably between 85° and 95° and most preferred 90°.

2. Tubing connector according to claim 1, wherein the connector comprises said first and a second liquid pressure measurement chamber, said second chamber comprising a second flexible membrane,
wherein the first chamber comprises the inlet and the first tubing connector element and
wherein the second chamber comprises the blood outlet and the second tubing connector element and wherein the membranes of the first and second pressure measurement chambers are arranged in the same plane or in parallel planes.

3. Tubing connector according to claim 2, wherein when the tubing connector is in a position with the plane of the membranes being vertical, the first chamber and the second chamber are located at the same vertical position or are offset from each other in the vertical direction.

4. Tubing connector according to claim 3 wherein each center of the two membranes is arranged in the direction of the one of the third and the fourth longitudinal axis.

5. Tubing connector according to according to any of the proceeding claim wherein at least one or both of the first and second longitudinal axes extend parallel to the plane of at least one or both of the two membranes.

6. Tubing connector according to any one of claims 1 to 5, wherein the first longitudinal axis and the second longitudinal axis lie on the same straight line or lie on straight lines which are parallel to each other or form an angle with each other which is in the range of one of the ranges of between 75° and 150°, between 75° and 105°, between 85° and 140°, between 85° and 95°, or in particular 90° or 135° and / or the third longitudinal axes and fourth longitudinal axis form an angle with each other which is in the range between 75° and 105°, preferably between 85° and 95° and most preferred 90°.

7. Tubing connector according to any one of claims 2 to 6, wherein the tubing connector comprises a further fluid inlet having a channel with an opening into the first or the second pressure measuring chamber, wherein preferably the tubing connector comprises a groove which is configured to hold a tubing which is configured to be fluidly connected to the further fluid inlet.

8. Tubing connector according to claim 7, wherein the further fluid inlet is located separate from the inlet and/or the outlet of the tubing connector or wherein the further fluid inlet and one of the inlet and the outlet of the tubing connector are formed by a common element.

9. Tubing connector according to claim 7 or 8, wherein a fifth longitudinal axis of the channel of the further fluid inlet is parallel to the second and third longitudinal axis, and preferably the fifth, the second and the third longitudinal axis lie in a same plane.

10. Tubing connector according to any one of claims 1 to 9, wherein the longitudinal axis of at least one of the inlet, the outlet, the first tubing connector element and the second tubing connector extends in an angle with respect to a plane of the membrane, in particular in an angle in the range from 30° to 60°, more preferably in the rage from 40° to 50° and most preferred in an angle of 45°.

11. Tubing connector according to any one the preceding claims, wherein the first membrane of the at least a first pressure measurement chamber is recessed with respect to an outer side of the pressure measurement chamber.

12. Tubing connector according to any one of the preceding claims, wherein the first pressure measurement chamber comprises a fixing element for fixing the first membrane to the connector or wherein the membrane is fixed to the first chamber without a fixing element.

13. Tubing connector according to any one of the preceding claims wherein the first pressure measurement chamber comprises a circumferential edge, in particular a circular ring, over which the first membrane is stretched.

14. Tubing connector according to any one of the preceding claims, wherein the at least one of the inlet, the outlet, the first tubing connector element and the second tubing connector element are arranged with relation to the membrane so that the first membrane is not directly exposed to flow of blood which enters the first pressure measurement chamber.

15. Tubing connector according to any one of the preceding claims, wherein the first pressure measurement chamber comprises a deflector that prevents direct flow of blood to the first membrane.

16. Tubing connector according to claim 2, wherein the second pressure measurement chamber and/or the second membrane is configured according to any one of claims 3 to 14.

17. Tubing connector according to claim 15, wherein the first and second membranes are formed by separate membranes and/or wherein the first and second membranes have identical dimensions and in particular are identical.

18. Tubing connector according to any one of the preceding claims, wherein the tubing connector comprises a push tab for applying a force in order to insert the tubing connector into a pump bed arrangement.

19. Tubing connector according to any one the preceding claims, wherein the first and/or the second pressure measurement chamber comprises one or more guide plates, preferably exactly one guide plate in a chamber.

20. Tubing connector according to claim 19, wherein the guide plate is fin-shaped.

21. Tubing connector according to claim 19 or 20, wherein the guide plate extends in a direction of an imaginary line which connects the inlet of the connector with the first tubing connector and/or which connects the second tubing connector with the outlet of the connector.

22. Tubing connector according to any one of claims 19 to 21, wherein the guide plate extends from the bottom of the pressure measurement chamber to the respective flexible membrane, wherein preferably the guide plate does not touch the membrane in normal operation of the tubing connector.

23. Tubing connector according to any one of claims 19 to 22, wherein the guide plate has a longitudinal cross section in form of a triangle, preferably in form of triangle with a rounded tip and/or wherein the guide plate is linear in cross-section.

24. Tubing connector arrangement comprising a tubing connector according to any one of the preceding claims, wherein a pump tubing is connected to the first tubing connector element and the second tubing connector element and/or a first tubing is connected to the inlet and a second tubing is connected to the outlet

25. Tubing connector arrangement according to claim 24, wherein the first tubing comprises a first branch tubing and the second tubing comprises a second branch tubing, wherein the first branch tubing and the second branch tubing are connected to each other via a connection portion and, wherein on the tubing connector arrangement only one pump-tubing is provided.

26. Tubing connector arrangement according to any one of claim 24 or 25, wherein the tubing connector comprises a first pressure measurement chamber being fluidly connected to the inlet and the first tubing connector and a second pressure measurement chamber being fluidly connected to the outlet and the second tubing connector.

27. Blood treatment device, comprising:
a pump, preferably a roller pump for pumping a liquid, preferably blood,
a tubing connector arrangement according to any one of claims 24 to 26,
wherein the pump tubing fluidly connects the roller pump with the tubing connector, wherein the tubing is at least in part is in contact with the roller pump, so that when the roller pump is rotating a liquid is conveyed through the pump tubing.
